# EUROPEAN PATENT APPLICATION

(11) **EP 4 613 196 A1**
(43) Date of publication of application: **10.09.2025**
(21) Application number: 25161284.2
(22) Date of filing: 03.03.2025
(51) Int. Cl.: A61B 5/15, A61B 5/151

(54) **LANCET FOR USE WITH A DERMAL PATCH SYSTEM**

(30) Priority: 06.03.2024 US 202418597513
(71) Applicant: Satio, Inc., Boston, Massachusetts 02210 (US)
(72) Inventor: Nawana, Namal, Weston, MA 02493 (US); Al-Shamsie, Ziad Tarik, San Diego, CA 92103 (US); Moniz, Mike, Boston, MA 02210 (US)
(74) Representative: Grünecker Patent- und Rechtsanwälte PartG mbB

(57) **Abstract**

A dermal patch system includes a lancet with three needles and a cartridge that is configured to attach to the skin of a subject. The lancet is configured automatically move the three needles from an undeployed position to a deployed position when the lancet engages with the cartridge. The three needles are configured to puncture the skin of the subject to draw a physiological sample when in the deployed position.

## Description

### RELATED APPLICATIONS

The present application claims priority to U.S. Application No. 17/719,881 filed on April 13, 2022, which claims priority to U.S. Provisional Patent Application No. 63/174,956 filed on April 14, 2021, U.S. Application No. 17/412,205 filed on August 25, 2021, which claims priority to U.S. Provisional Patent Application No. 63/190,700 filed on May 19, 2021 and U.S. Provisional Patent Application No. 63/174,956 filed on April 14, 2021, U.S. Application No. 17/747,544 filed on May 18, 2022, which claims priority to U.S. Provisional Patent Application No. 63/190,700 filed on May 19, 2021, U.S. Application No. 17/903,802 filed on September 6, 2022, U.S. Application No. 18/090,026 filed on December 28, 2022, U.S. Application No. 17/971,142 filed on October 21, 2022, U.S. Application No. 17/991,284 filed on November 21, 2022 and U.S. Application No. 18/090,063 filed on December 28, 2022, which are hereby incorporated by reference herein in their entireties.

### TECHNICAL FIELD

The present teachings are generally directed to a lancet for use with a cartridge of a dermal patch system (also referred to as "a dermal patch" herein).

### BACKGROUND

Several diagnostic tests may be performed by analyzing a physiological sample from a subject. In order to obtain the physiological sample, a subject may have to travel to a diagnostic lab, where a medical professional obtains the sample with a standard syringe. This process may be time consuming and burdensome to the subject.

### SUMMARY

Aspects of the present disclosure address the above-referenced problems and/or others.

In one aspect, a dermal patch system includes a lancet including one, two or three or four, or more needles, and a cartridge configured to attach to the skin of a subject. The lancet is configured automatically move the needle(s) from an undeployed position to a deployed position when the lancet engages with the cartridge. The needle(s) are configured to puncture the skin of the subject to draw a physiological sample when in the deployed position. In some embodiments, the cartridge is configured to store the drawn physiological sample. In some embodiments, when three needles are used, the three needles can be equally spaced from one another. In these embodiments, tips of the three needles form an equilateral triangle. In some embodiments, the lancet includes a housing, and the needle(s) are disposed within the housing when in the undeployed position and extend beyond the housing when in the deployed position. In some embodiments, the needle(s) are configured to automatically retract into the housing after puncturing the skin. In some embodiments, the lancet further includes: an injection spring configured to move the needle(s) to the deployed position, and a retraction spring configured to retract the needle(s) into the housing. In some embodiments, the lancet includes a mechanism for maintaining the retraction spring is a compressed state when the needle(s) are in the undeployed position and is configured to expand to retract the needle(s). In some embodiments, the lancet further includes a plurality of deformable tabs configured to compress the retraction spring when the needle(s) are in the undeployed position. In some embodiments, the lancet further includes a needle platform that carries the needle(s) from the unemployed position to the deployed position. The needle platform is configured to deform the tabs and, when deformed, the tabs allow the retraction spring to expand. In some embodiments, the needle platform is configured to break the tabs.

In another aspect, a method for obtaining a physiological sample from a subject includes affixing a cartridge of a dermal patch system to the skin of a subject, and engaging a lancet with the cartridge, wherein the lancet includes needle(s). Engaging the lancet with the cartridge automatically moves the needle(s) from an undeployed position to a deployed position thereby drawing a physiological sample. In some embodiments, the method also includes storing physiological sample within the cartridge. In some embodiments, three needles are equally spaced from one another. In some embodiments, the tips of the three needles form an equilateral triangle. In some embodiments, the method also includes retracting the needle(s) into the lancet, and removing the cartridge from the skin of the subject. In some embodiments, the lancet automatically retracts the needle(s). In some embodiments, the lancet includes a first spring that moves the needle(s) to the deployed position and a second spring that retracts the needle(s). In some embodiments, the method also includes deforming a portion of the lancet whole moving needle(s) to the deployed position. In another embodiments, the method also includes deforming a portion of the lancet allows the second spring to retract the needle(s).

### BRIEF DESCRIPTION OF THE DRAWINGS

Aspects of the present disclosure may take form in various components and arrangements of components, and in various steps and arrangements of steps. The drawings are only for illustration purpose of preferred embodiments of the present disclosure and are not to be considered as limiting.

Features of embodiments of the present disclosure will be more readily understood from the following detailed description take in conjunction with the accompanying drawings in which:
**Fig. 1** depicts a dermal patch system in accordance with an exemplary embodiment of the present disclosure;
**Fig. 2** depicts a lancet with three needles in accordance with an exemplary embodiment of the present disclosure;
**Fig. 3** is a cross sectional view of the lancet, wherein the lancet is in an undeployed position in accordance with an exemplary embodiment of the present disclosure;
**Fig. 4** is a cross sectional view of a housing of the lancet in accordance with an exemplary embodiment of the present disclosure;
**Fig. 5** is a cross sectional view of a cap of the lancet in accordance with an exemplary embodiment of the present disclosure;
**Fig. 6** depicts a side of an inner sleeve of the lancet in accordance with an exemplary embodiment of the present disclosure;
**Fig. 7** depicts another side of the inner sleeve of the lancet in accordance with an exemplary embodiment of the present disclosure;
**Fig. 8** is a cross sectional view of the inner sleeve of the lancet in accordance with an exemplary embodiment of the present disclosure;
**Fig. 9** depicts a side of a needle platform of the lancet in accordance with an exemplary embodiment of the present disclosure;
**Fig. 10** depicts another side of the needle platform in accordance with an exemplary embodiment of the present disclosure;
**Fig. 11** is a cross sectional view of a spring sleeve of the lancet in accordance with an exemplary embodiment of the present disclosure;
**Fig. 12** is a top view of a cover of a cartridge of the dermal patch system in accordance with an exemplary embodiment of the present disclosure;
**Fig. 13** is a cross sectional view of the cover in accordance with an exemplary embodiment of the present disclosure;
**Fig. 14** is a top view of a base of the cartridge of the dermal patch system in accordance with an exemplary embodiment of the present disclosure;
**Fig. 15** is a cross sectional view of a lancet receiving element of the base in accordance with an exemplary embodiment of the present disclosure;
**Fig. 16** is a cross sectional view of the base in accordance with an exemplary embodiment of the present disclosure;
**Fig. 17** schematically depicts a physiological sample well, a physiological sample channel, and a sample collection reservoir of the base in accordance with an exemplary embodiment of the present disclosure;
**Fig. 18** is a cross sectional view of the lancet engaged with the base, wherein the needle platform is in a released position in accordance with an exemplary embodiment of the present disclosure;
**Fig. 19** is a cross sectional view of the lancet, wherein the needle platform is in a released position in accordance with an exemplary embodiment of the present disclosure;
**Fig. 20** is a cross sectional view of the lancet transitioning to a deployed position in accordance with an exemplary embodiment of the present disclosure;
**Fig. 21** is a cross sectional view of the lancet engaged with the base, wherein the lancet is in a deployed position in accordance with an exemplary embodiment of the present disclosure;
**Fig. 22** is a cross sectional view of the lancet, wherein the lancet is in a deployed position in accordance with an exemplary embodiment of the present disclosure;
**Fig. 23** is a cross sectional view of the lancet engaged with the base, wherein the lancet is in a retracted position in accordance with an exemplary embodiment of the present disclosure;
**Fig. 24** is a cross sectional view of the lancet, wherein the lancet is in a retracted position in accordance with an exemplary embodiment of the present disclosure;

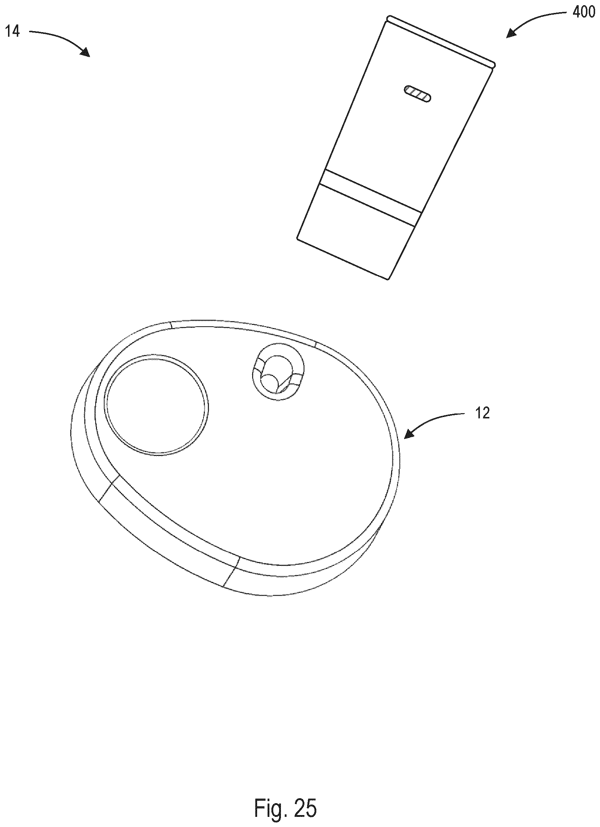

**Fig. 25** depicts a dermal patch system in accordance with an exemplary embodiment of the present disclosure;
**Fig. 26** shows the lancet with a sensor formed therein;

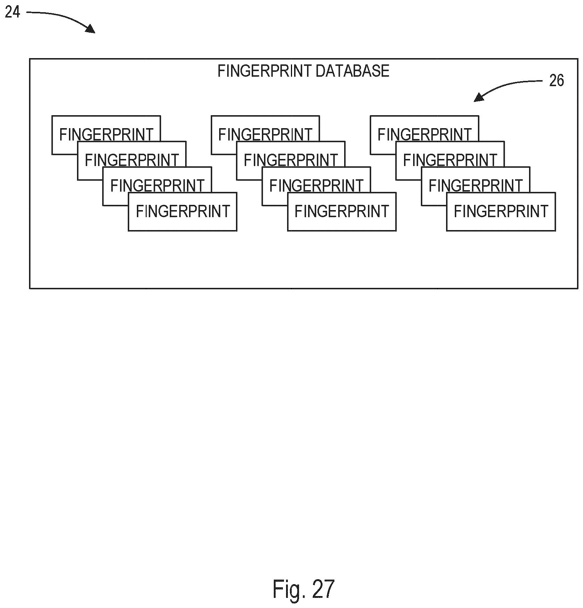

**Fig. 27** shows a fingerprint database in accordance with an exemplary embodiment of the present disclosure;
**Fig. 28** is a cross sectional view of the lancet, wherein the lancet is in a fully retracted position in accordance with an exemplary embodiment of the present disclosure;
**Fig. 29** is a cross sectional view of the lancet, wherein the lancet is in a fully retracted position in accordance with an exemplary embodiment of the present disclosure;
**Fig. 30** shows a computer system connected to a fingerprint data base in accordance with an exemplary embodiment of the present disclosure;
**Fig. 31** shows a computer system connected to a biometric data base in accordance with an exemplary embodiment of the present disclosure;
**Fig. 32** shows a computer system connected to an EMR data base in accordance with an exemplary embodiment of the present disclosure;
**Fig. 33** shows a method for obtaining a physiological sample from a subject in accordance with an exemplary embodiment of the present disclosure;
**Fig. 34** is a flow chart showing a method for obtaining a physiological sample from a subject in accordance with an exemplary embodiment of the present disclosure;
**Fig. 35** shows a computer system in accordance with an exemplary embodiment of the present disclosure;
**Fig. 36** shows a cloud computing environment in accordance with an exemplary embodiment of the present disclosure.

### DETAILED DESCRIPTION

The present disclosure generally relates to a dermal patch system (which may also be referred to as a "dermal patch") that may be utilized to collect and optionally store a physiological sample.

In various aspects, the present teachings address an unmet need for devices that allow efficient, easy and reliable extraction of biological samples from a subject.

In various embodiments, a dermal patch system is disclosed that allows collecting a physiological sample and storing the collected physiological sample, e.g., on a sample collection pad and/or a reservoir provided on the dermal patch system. Dermal patch systems disclosed herein may allow for the collection and analysis of a physiological sample in a variety of environments (e.g., at home, in the field, in a medical facility, etc.).

The term "about," as used herein, denotes a deviation of at most 10% relative to a numerical value. For example, about 100 µm means in the range of 90 µm-110 µm.

The term "substantially," as used herein, refers to a deviation, if any, of at most 10% from a complete state and/or condition.

The term "majority," as used herein, refers to more than 50% of an object or substance.

The term "subject" as used herein refers to a human subject or an animal subject (i.e., chicken, pig, cattle, dog, cat, etc.).

The term "transparent," as used herein, indicates that light can substantially pass through an object (e.g., a window) to allow visualization of a material disposed behind the object. For example, in some embodiments, a transparent object allows the passage of at least 70%, or at least 80%, or at least 90% of visible light therethrough.

The term "needle" as used herein, refers to a component with a pointed tip that is configured to pierce an outer surface of an element (e.g., skin of a subject) to provide a passageway through which a physiological fluid can be extracted. One, two, three, four, or more needles can be used.

The present disclosure generally relates to a device, which is herein also referred to as a dermal patch or a dermal patch system, for collecting a physiological sample (e.g., bodily fluids such as blood, interstitial fluids, etc.) from a subject. In some embodiments discussed below, such a dermal patch system can include a cartridge that can be affixed to a subject's skin (e.g., via an adhesive layer) and a separate lancet that can be engaged with the cartridge to puncture the skin, thereby providing a passageway for extracting the physiological sample. As discussed in more detail below, the lancet can include a housing in which at least one needle that is configured for puncturing the skin is disposed. The lancet can be transitioned between at least three states. In a first state (which may be referred to as an undeployed or a locked state) the lancet retains the needle(s) within the lancet housing when the lancet is not engaged with the cartridge. In a second state (which may be referred to as a deployed, an unlocked state, or a released state) the lancet allows the needle(s) to be deployed to allow the needle(s) to exit the housing, so as to be available for puncturing the skin in response to engagement of the lancet with the cartridge. In a third state (which may be referred to as a retracted state), subsequent to the deployment of the needle(s), the lancet automatically moves the needle(s) from the deployed position back into the lancet housing. In other words, the engagement of the lancet with the cartridge automatically transitions the needle(s) from the locked state to the unlocked state, and to the retracted state.

In some embodiments, as discussed in more detail below, the lancet includes an inner sleeve with a plurality of deformable tabs and a spring sleeve. In a locked state, the deformable tabs can apply a pressure to the spring sleeve to maintain the spring sleeve in a first position in which the spring sleeve compresses the lower spring into a compressed state and prevents the lower spring from expanding.

When the lancet is engaged with the cartridge, the inner sleeve moves from a first position to a second position. In the first position, the inner sleeve retains a needle platform that supports the needle(s) in an undeployed position. The lancet also includes an upper spring that is coupled to the needle platform. In the undeployed position, the needle platform compresses the upper spring and prevents the upper spring form expanding. In the second position, the inner sleeve moves vertically upward, which releases the needle platform thereby allowing the needle platform to move to a deployed position. When released, the upper spring is allowed to expand thereby applying a force to the needle platform, which causes the movement of the needle platform such that the needle(s) extend beyond the housing of the lancet and become available for puncturing the skin.

Furthermore, this force causes the needle(s) (which are coupled to the needle platform) to travel with sufficient velocity to puncture the skin of a subject. As the needle platform travels to the deployed position, the needle platform contacts the deformable tabs and causes the tabs to break or deflect. When broken (or deflected), the tabs no longer contact the spring sleeve, or apply a lower force onto the spring sleeve, thereby allowing the lower spring to expand and move the spring sleeve vertically upward from a first position to a second position, which in turn moves the needle spring vertically upward such that the needle(s) are retracted into the housing of the lancet.

In this manner, the lancet remains safe before it is engaged with the cartridge as the needle(s) in the lancet cannot be deployed when the lancet is not engaged with the cartridge. Furthermore, in this manner, the lancet remains safe after drawing a physiological sample as the needle(s) automatically retract back into the lancet's housing after being deployed.

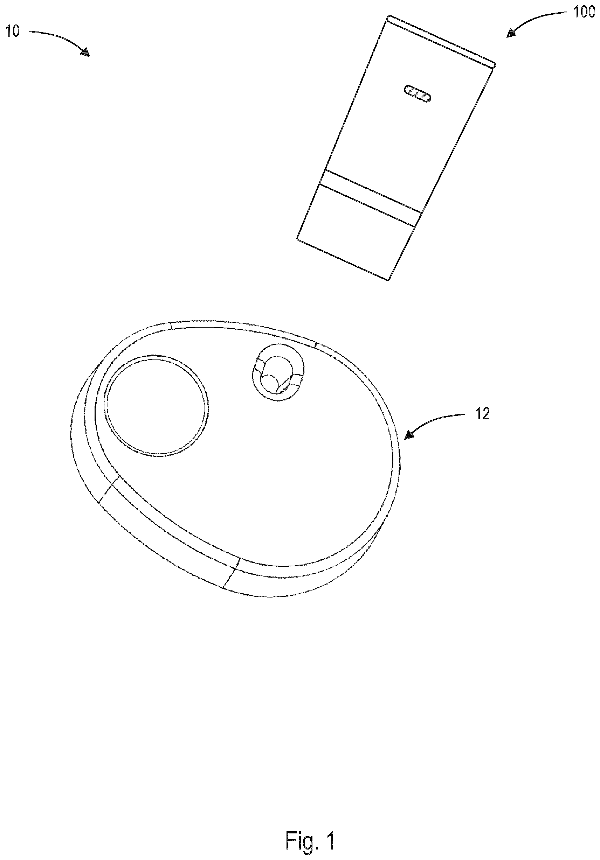

Referring now to **Fig. 1****,** a dermal patch system **10** is shown in accordance with an exemplary embodiment. In this embodiment, the dermal patch system **10** includes a cartridge **12** that can be affixed to a subject's skin and a lancet **100** that can be engaged with the cartridge **12.**

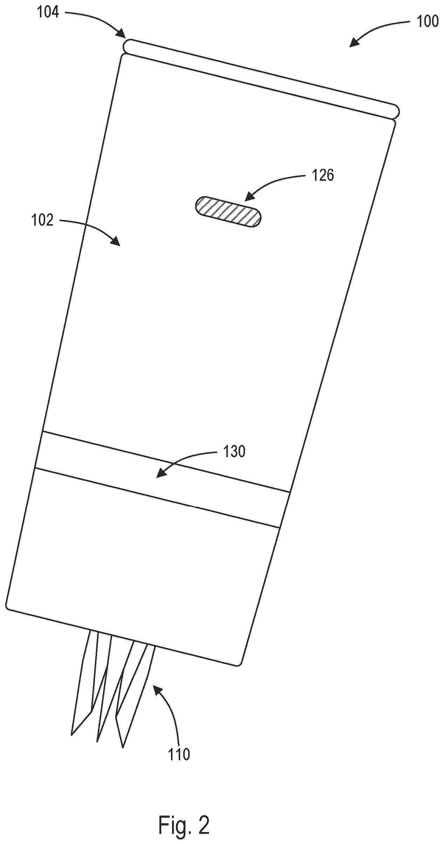

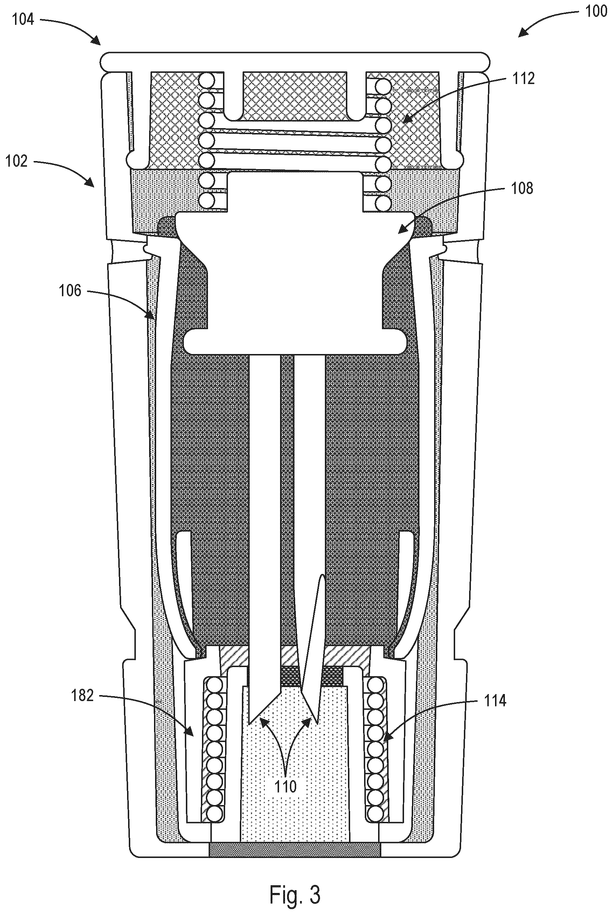

Referring to **Figs. 2** **and** **3****,** a cross sectional view of the lancet **100** is shown in accordance with an exemplary embodiment. The lancet **100** include a housing **102** and a cap **104** that is coupled to the housing **102.** The lancet extends longitudinally along an X-axis **(****Fig. 3****)** between a proximal end defined by the cap **104** and a distal end defined by the housing **102.**

The housing **102** and the cap **104** define an inner volume of the lancet **100** in which various components of the lancet **100** are disposed. The lancet **100** further includes an inner sleeve 106 and a needle platform **108** that are disposed within the inner volume of the lancet **100.** The needle platform **108** supports a plurality of needles **110,** specifically three needles **110** in this embodiment. Although three needles are shown in Figs. 2 and 3, as previously mentioned, one, two, three or four, or more needles can be used.

While needle platform **108** is depicted as supporting three needles **110 (****Fig. 2****),** in other embodiments, the needle platform **108** can support more or less needles **110** (e.g., 1, 2, 4, 5, needles etc.). However, it has been discovered that the use of three needles is particularly advantageous in drawing blood from a subject. Further, in this embodiment, the needles have slanted ends and the bottom ends of the needles facing away from one another.

The lancet **100** further includes an injection spring (also referred to as an upper spring) **112** and a retraction spring (also referred to as a lower spring) **114** that can move the needle platform **108.**

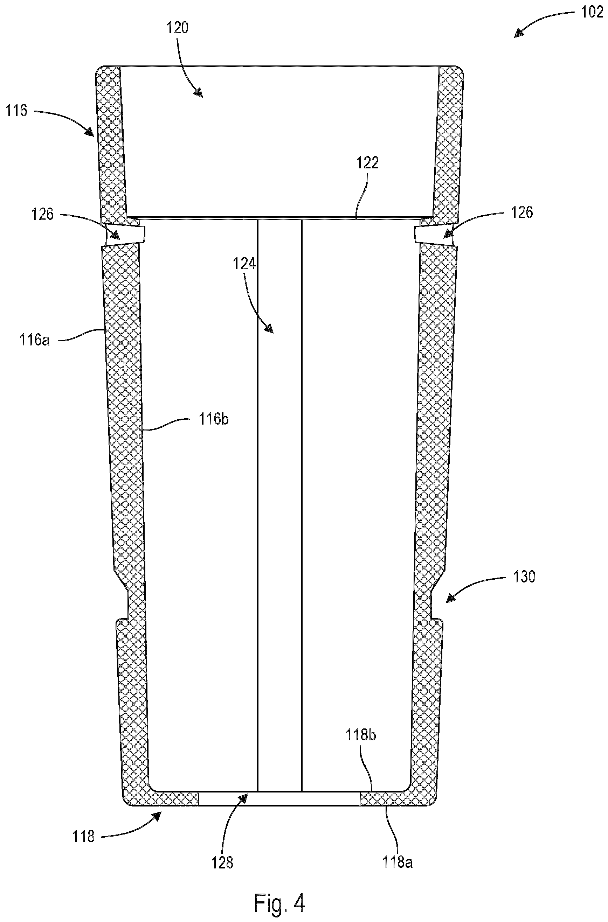

With particular reference to **Fig 4****,** the housing **102** includes a side wall **116** and a bottom wall **118.** The side wall **116** includes an outer surface **116a** and an opposed inner surface **116b.** The bottom wall **118** includes an outer surface **118a** and an opposed inner surface **118b.** The side wall **116** extends vertically from the bottom wall **118.** In this embodiment, the side wall **116** is substantially cylindrical in shape and the bottom wall **118** has a substantially circular cross-section. The side wall **116** and the bottom wall **118** are generally symmetric about a longitudinal axis (Y-axis) of the lancet **100.**

The inner surface **116b** of the side wall **116** and the inner surface **118b** of the bottom wall **118** define an inner volume **120.** The side wall **116** is tapered such that its inner diameter increases in a direction from its distal end to its proximal end such that the generally the lancet housing exhibits a wider upper portion relative to its lower portion. Further, the inner surface **118b** of the upper portion defines a ledge **122.**

The inner surface **116b** also defines a first and second longitudinal groove **124** that are opposed from one another (only one of which is shown in the cross section in **Fig. 4****).** The grooves **124** extend between the ledge **122** and the inner surface **118b** of the bottom wall **118.**

The side wall **116** defines openings **126** that extend through the side wall **116.** That is, the openings **126** extend between the outer surface **116a** and the inner surface **116b** of the side wall **116.** The bottom wall **118** defines an aperture **128** that extends through the bottom wall **118.** That is, the aperture **128** extends between the outer surface **118a** and the inner surface **118b** of the bottom wall **118.** As will be discussed in further detail herein, when the lancet **100** is activated via engagement with the cartridge **12,** the needle(s) of the lancet **100** extend through the aperture **128** for puncturing a subject's skin to draw a physiological sample. The outer surface **116b** of the side wall **116** further defines an outer groove **130.** As will be discussed in further detail herein, a portion of the cartridge **12** engages with the outer groove **130** to couple the lancet **100** to the cartridge **12.**

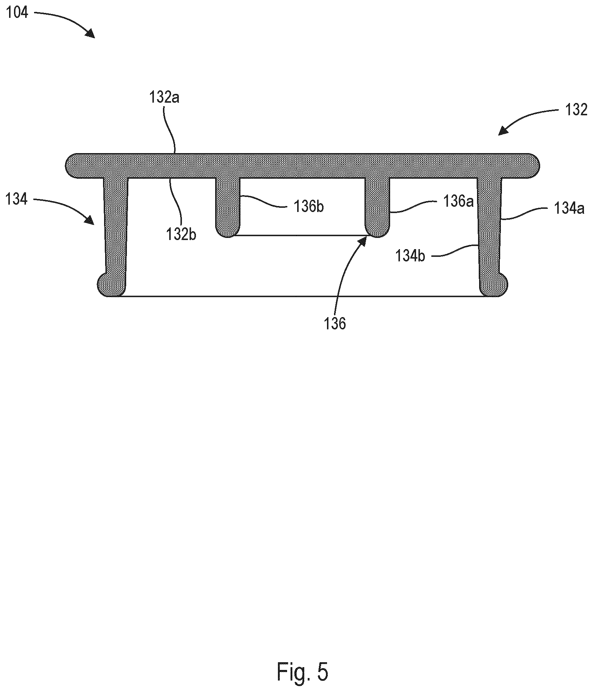

Referring now to **Fig. 5****,** the inner cap **104** is shown in accordance with an exemplary embodiment. The cap **104** includes a top wall **132** with an outer surface **132a** and an opposed inner surface **132b.** The cap **104** also includes a side wall **134** with an outer surface **134a** and an opposed inner surface **134b.** The top wall **132** extends substantially horizontally from and perpendicular to the side wall **134.** The side wall **134** extends substantially vertically from and perpendicular to the top wall **132.** The top wall **132** and the side wall **134** have generally circular cross-sectional profiles and are concentric with one another. The cap **104** also includes an inner cylinder **136** with an outer surface **136a** and an opposed inner surface **136b.** The inner cylinder **136** extends vertically from and perpendicular to the top wall **132.** The inner cylinder **136** is generally concentric with the top wall **132** and the side wall **134.** The inner cylinder **136** extends substantially vertically from and perpendicular to the top wall **132.**

When the cap **104** is coupled to the housing **102** the side wall **134** extends into the inner volume **120** of the housing **102** and at least a portion of the side wall **134** contacts the inner surface **116b** of the side wall **116** such that the cap **104** couples to the housing **102** via an interference fit.

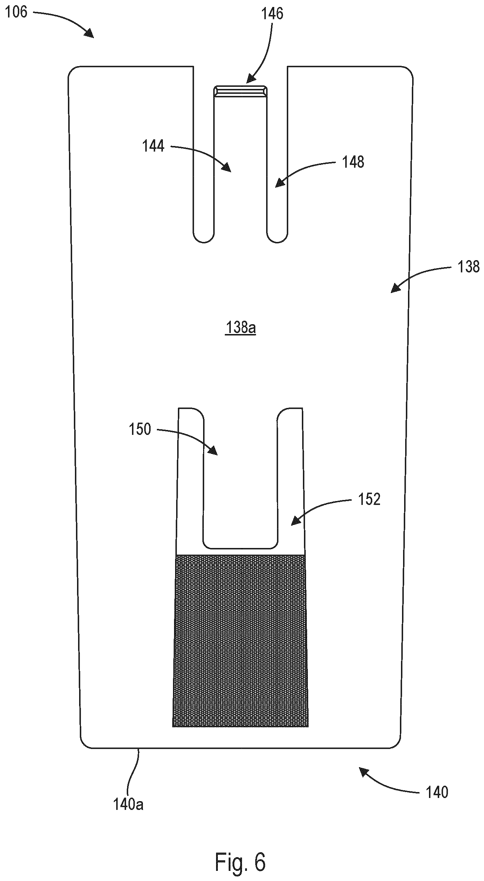

Referring now to **Figs. 6-8****,** the inner sleeve **106** is shown in accordance with an exemplary embodiment. The inner sleeve **106** includes a side wall **138** and a bottom wall **140.** The side wall **138** includes an outer surface **138a** and an opposed inner surface **138b.** The bottom wall **140** includes an outer surface **140a** and an opposed inner surface **140b.** The side wall **138** extends substantially vertically from the bottom wall **140.** The side wall **138** and the bottom wall **140** have generally circular cross-sectional profiles and are positioned concentrically relative to one another. The inner surface **138b** of the side wall **138** and the inner surface **140b** of the bottom wall **140** define an inner volume **142.**

The side wall **138** defines a first and second deformable extension **144** that extend within a gap **148** of the side wall **138.** The extensions **144** each include a hook **146.** As will be discussed in further detail herein, the hooks **146** of the extensions **144** can be received by the openings **126** of the housing **102** to place which the lancet **100** in the undeployed position.

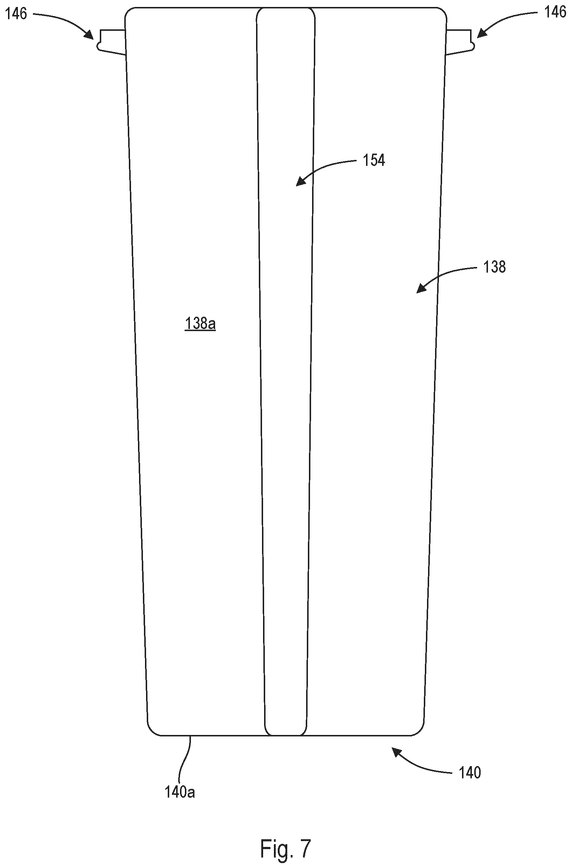

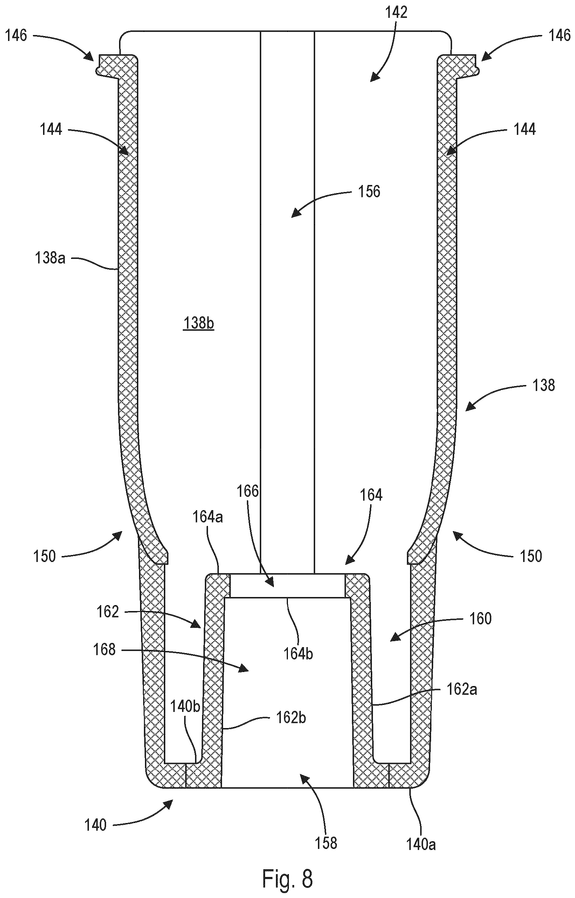

The side wall **138** further defines a first and second deformable tabs **150** that extend within an opening **152** of the side wall **138.** The side wall **138** also includes a first and second protrusions **154** (only one of which is shown in **Fig. 7****)** that extend substantially horizontally from and perpendicular to the outer surface **138a** of the side wall **138.** When the inner sleeve **106** is disposed within the housing **102,** the protrusions **154** extend within the grooves **124** of the housing **102.** When the inner sleeve **106** is moving between different positions (e.g., between an undeployed position to a deployed position), the grooves **124** and the protrusions **154** guide the vertical movement of the inner sleeve **106** while preventing a rotational or horizontal movement of the inner sleeve **106.** The inner surface **138b** of the side wall **138** further defines a first and second grooves **156** that are opposed from one another (only one of which is shown in **Fig. 8****).** The grooves **156** extend substantially vertically from and perpendicular to the inner surface **140b** of the bottom wall **140.**

The inner sleeve **106** includes an opening **158** that extends through the bottom wall **140.** That is, the opening **158** extends between the outer surface **140a** and the inner surface **140b** of the bottom wall **140.** The inner sleeve **106** also includes a post **160** that is cylindrical in shape and is generally concentric with the opening **158.** The post **160** includes a side wall **162** and a top wall **164.** The side wall **162** extends substantially vertically from and perpendicular to the inner surface **140b** of the bottom wall **140.** The side wall **162** includes an outer surface **162a** and an opposed inner surface **162b.** The top wall **164** extends substantially horizontally between the side wall **162** and includes a top surface **164a** and an opposed bottom surface **164b.** The top wall **164** includes an opening **166** that extends through the top wall **164.** That is, the opening **166** extends between the top surface **164a** and the bottom surface **164b** of the top wall **164.** The inner surface **162b** of the side wall **162** and the bottom surface **164b** of the top wall **164** define an inner volume **168** of the post **160.** As will be discussed in further detail herein, the needle(s) **110** extend through the opening **166** of the top wall **164,** through the inner volume **168,** and through the opening **158** of the bottom wall **140** when in the deployed position.

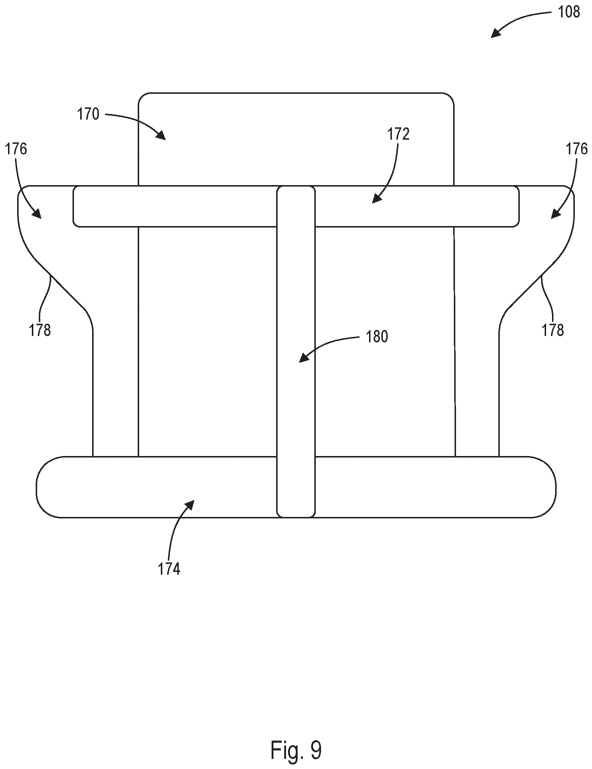

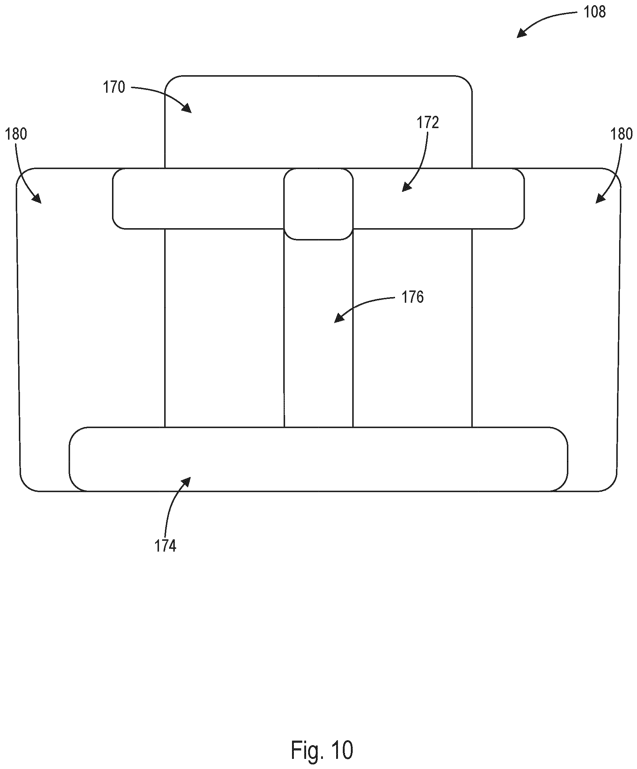

Referring now to **Figs. 9** **and** **10****,** the needle platform **108** is shown in accordance with an exemplary embodiment. The needle platform **108** includes a cylindrical body **170,** an upper circular extension **172** and a lower circular extension **174.** The upper circular extension **172** and the lower circular extension **174** extend substantially horizontally from and perpendicular to the cylindrical body **170.** As will be discussed in further detail herein, the upper circular extension **172** serves as a platform for the injection spring **112.** The needle platform **108** further includes a first and second curved extensions **176.** The curved extensions **176** extend substantially horizontally from and perpendicular to the cylindrical body **170** and extend substantially vertically between the upper circular extension **172** and the lower circular extension **174.** Each curved extension **176** includes an angled surface **178.** As will be discussed in further detail herein, when the needle platform is in the undeployed position, the angled surfaces **178** contact the hooks **146** of the inner sleeve 106, which prevents the needle platform **108** from transitioning to the deployed position.

The needle platform **108** further includes first and second projections **180.** The first and second projections **180** are generally rectangular in shape, extend substantially horizontally from and perpendicular to the cylindrical body **170,** and extend substantially vertically between the upper circular extension **172** and the lower circular extension **174.** When the needle platform **108** is disposed within the inner sleeve **106,** the projections **180** extend into the grooves **156** which guides the vertical movement of the needle platform **108** while preventing a rotational or horizontal movement of the needle platform **108.**

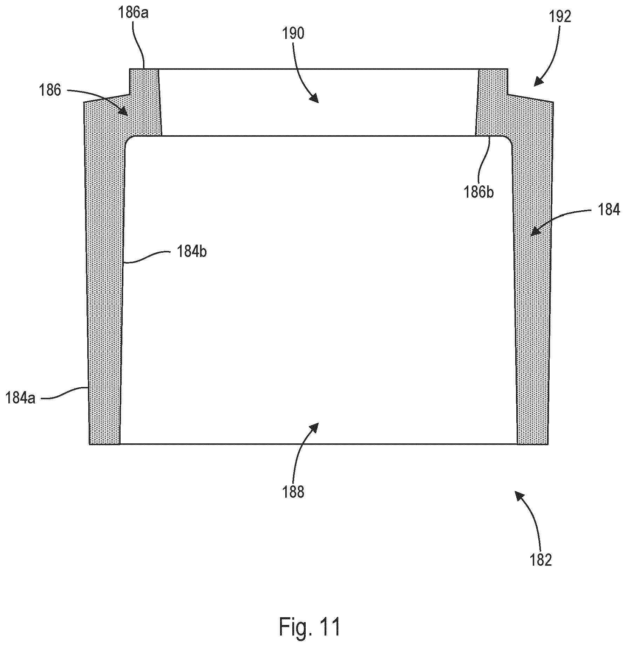

As depicted in **Fig. 3****,** the lancet **100** further includes a spring sleeve **182.** With particular reference to **Fig. 11****,** the spring sleeve **182** is shown in accordance with an exemplary embodiment. The spring sleeve **182** is cylindrical in shape and includes a side wall **184** and a top wall **186.** The side wall **184** includes an outer surface **184a** and an opposed inner surface **184b** and the top wall **186** includes a top surface **186a** and an opposed bottom surface **186b.** Th inner surface **184b** of the side wall **184** and the bottom surface **186b** of the top wall **186** define an inner volume **188** of the spring sleeve **182.** The side wall **184** and the top wall **186** are shaped and dimensioned such that the spring sleeve **182** retains the retraction spring **114** within the inner volume **188.** The top wall **186** includes an opening **190** that extends therethrough. That is, the opening **190** extends between the top surface **186a** and the bottom surface **186b** of the top wall **186.** The opening **190** of the top wall **186** is in communication with the inner volume **188.** Since the inner volume **188** is open, the needle(s) **110** may extend through spring sleeve **182** via the opening **190.** The top surface **184a** defines a ledge **192** that extends circumferentially around the spring sleeve **182.** As will be discussed in further detail herein, when the lancet **100** is in the undeployed position, the tabs **150** contact the ledge **192.**

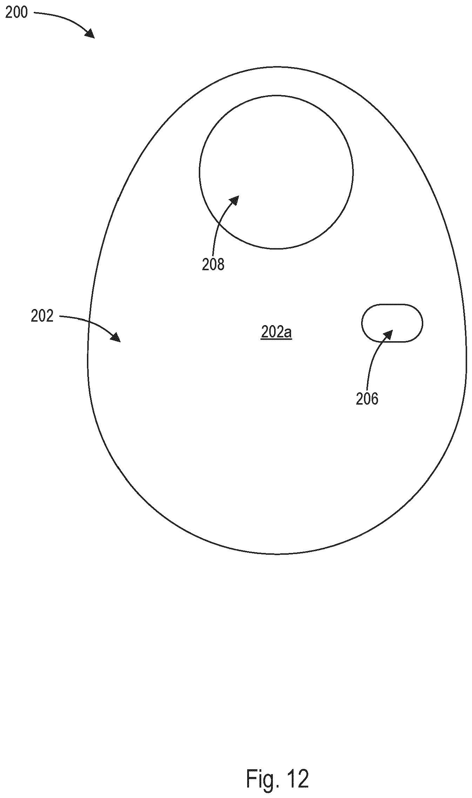

Referring now to **Figs. 12-17****,** the cartridge **12** is shown in accordance with an exemplary embodiment. The cartridge **12** is configured to attach to the skin of a subject and includes an adhesive layer (not shown) disposed on a bottom surface of the cartridge **12.** After use, the cartridge **12** may be removed by pulling the cartridge **12** off the skin of the subject. The cartridge **12** includes a cover **200** and a base **300** that can couple to the cover **200.** In one embodiment, the cover **200** and the base **300** are formed as two separate components that are removably coupled to one another (e.g., via a snap fitting). In another embodiment, the cover **200** and the base **300** form an integral unitary cartridge **12.** The cover **200** and the base **300** may be coupled to one another via an adhesive, laser welding, etc.

The cartridge **12** may be formed using a variety of suitable materials including, but not limited to, polymeric materials (e.g., polyolefins, polyethylene terephthalate (PET), polyurethanes, polynorbornenes, polyethers, polyacrylates, polyamides (Polyether block amide also referred to as Pebax^{®}), polysiloxanes, polyether amides, polyether esters, trans-polyisoprenes, polymethyl methacrylates (PMMA), cross-linked trans-polyoctylenes, cross-linked polyethylenes, cross-linked polyisoprenes, cross-linked polycyclooctenes, inorganic-organic hybrid polymers, co-polymer blends with polyethylene and Kraton^{®}, styrene-butadiene co-polymers, urethane-butadiene co-polymers, polycaprolactone or oligo caprolactone co-polymers, polylactic acid (PLLA) or polylactide (PL/DLA) co-polymers, PLLA-polyglycolic acid (PGA) co-polymers, photocross linkable polymers, etc.). In some embodiments, some of the cover 200 may be formed of poly(dimethylsiloxane) (PDMS) to allow visibility of components disposed within the cartridge **12.**

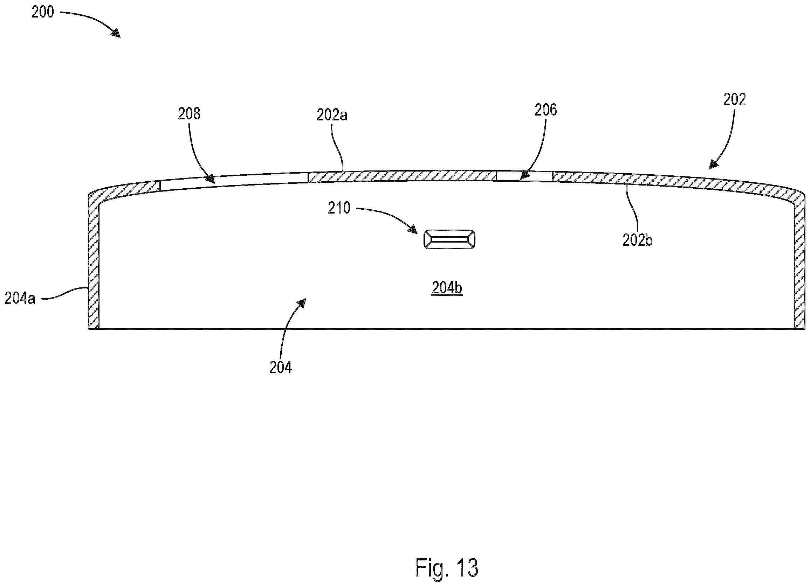

Referring now to **Figs. 12** **and** **13****,** the cover **200** is shown in accordance with an exemplary embodiment. The cover **200** includes a top wall **202** with an outer surface **202a** and an opposed inner surface **202b.** The cover **200** also includes a side wall **204** with an outer surface **204a** and an opposed inner surface **204b.** The top wall **202** extends substantially longitudinally from and perpendicular to the side wall **204.** The side wall **204** extends substantially vertically from and perpendicular to the top wall **202.**

The cover **200** includes a viewing aperture **206** that extends through the top wall **202.** That is, the viewing aperture **206** extends between the outer surface **202a** and the inner surface **202b** of the top wall **202.** In various embodiments, the viewing aperture **206** is covered by a transparent material, which allows a user of the dermal patch system to view components disposed within the cartridge **12.** The top wall **202** further includes a lancet aperture **208** that extends through the top wall **202.** That is, the lancet aperture **208** extends between the outer surface **202a** and the inner surface **202b** of the top wall **202.** The lancet aperture **208** is generally shaped and dimensioned to accept a portion of the lancet **100,** e.g., the aperture can be substantially circular. As will be discussed in further detail herein, a portion of the lancet **100** extends through the lancet aperture **208** to couple to the base **300.**

The cover **200** further includes a plurality of locking members **210** that extend substantially horizontally from and perpendicular to the inner surface **204a** of the side wall **204.** As will be discussed in further detail herein, the locking members **210** aid in coupling the cover **200** to the base **300.**

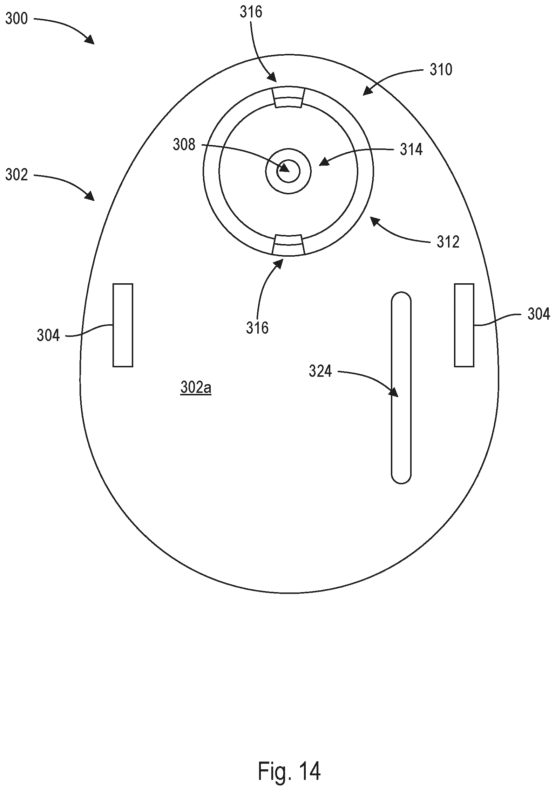

Referring now to **Figs. 14-17****,** the base **300** is shown in accordance with an exemplary embodiment. The base **300** includes a bottom wall **302** that includes a top surface **302a** and opposed bottom surface **302b.** The bottom wall **302** has a similar shape and dimension as the side wall **204** of the cover **200** such that the cover **200** and the base **300** are flush with one another when the cover **200** is coupled to the base **300.** Furthermore, when the cover **200** is coupled to the base **300,** the side wall **204** contacts the bottom wall **302.**

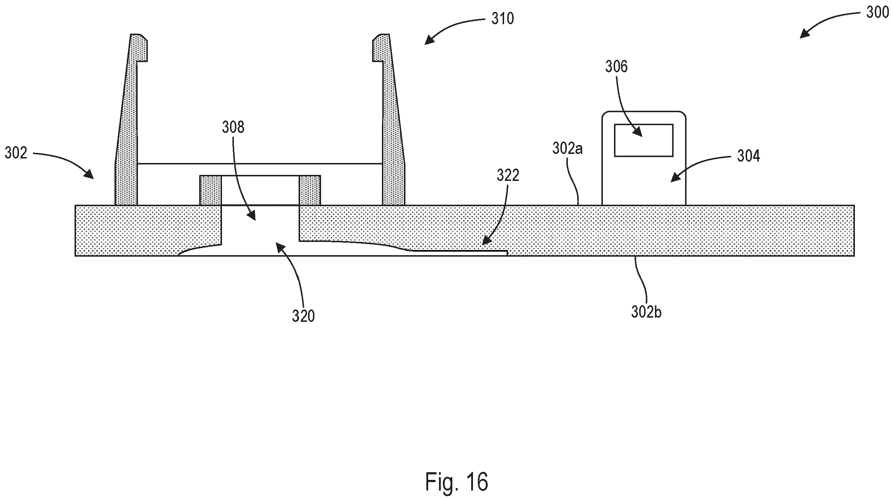

The base **300** includes a plurality of extensions **304** that extend substantially vertically from and perpendicular to the top surface **302a** of the bottom wall **302.** Each of the extensions **304** define a gap **306 (****Fig. 16****).** The gap **306,** and therefore the extensions **304,** are shaped and dimensioned to accept a locking member **210.** The locking members **210** extend through the gaps **306** to couple the cover **200** to the base **300.**

The base **300** further includes a needle aperture **308** that is generally circular in shape. The needle aperture **308** extends through the bottom wall **302.** That is, the needle aperture 308 extends between the top surface **302a** and the bottom surface **302b** of the bottom wall **302.** As will be discussed in further detail herein, when the cover **200** is coupled to the base **300** and when the cartridge **12** is adhered to a subject, the needle aperture **308** allows the needle(s) **110** of the lancet **100** to extend through the bottom wall **302** to puncture the subject's skin.

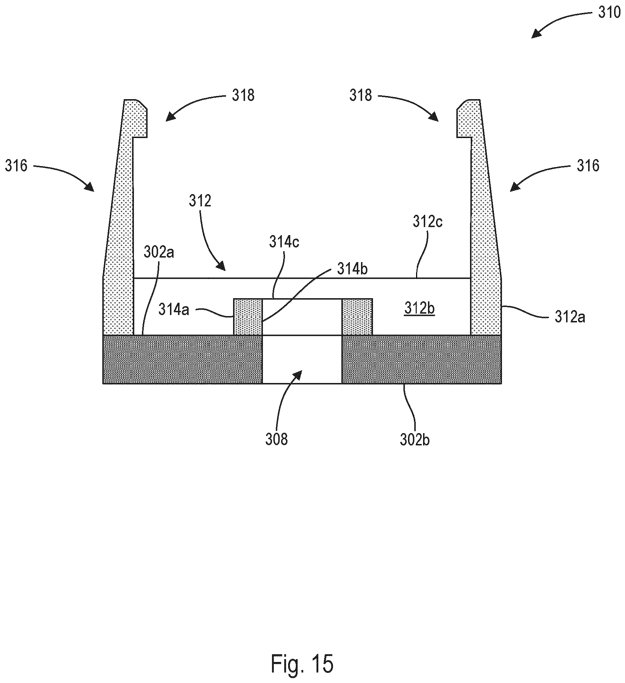

The base **300** also includes lancet receiving element **310** that is shaped and dimensioned to accept a distal end of the lancet **100.** With particular reference to **Fig. 15****,** the lancet receiving element **310** includes an outer circular projection **312** and an inner circular projection **314** (see **Fig. 14****),** each extending substantially vertically from and perpendicular to the top surface **302a** of the bottom wall **302.** The outer circular projection **312** includes an outer surface **312a,** an opposed inner surface **312b,** and a top surface **312c** that extends between the outer surface **312a** and the inner surface **312b.** The top surface **312c** extends substantially perpendicular to and horizontally between the outer surface **312a** and the inner surface **312b.** The outer surface **312a** and the inner surface **312b** extend substantially vertically from and perpendicular to the top surface **302a** of the bottom wall **302.** The outer circular projection **312** is shaped to accept the lancet **100.**

The inner circular projection **314** is disposed around the needle aperture **308** and includes an outer surface **314a,** an opposed inner surface **314b,** and a top surface **314c** that extends between the outer surface **314a** and the inner surface **314b.** The top surface **314c** extends substantially perpendicular to and horizontally between the outer surface **314a** and the inner surface **314b.** The outer surface **314a** and the inner surface **314b** extend substantially vertically from and perpendicular to the top surface **302a** of the bottom wall **302.** The outer circular projection **312** and the inner circular projection **314** circular projection are substantially concentric with one another.

As will be discussed in further detail herein, when the lancet **100** is engaged with the cartridge **12,** the top surface **314c** of the inner circular projection **314** contacts a portion of the lancet 100 which causes the lancet **100** to transition the needle(s) **110** from the undeployed position to the deployed position.

Withe continued reference to **Fig. 15****,** the lancet receiving element **310** further includes a plurality of locking members **316** that extend substantially vertically from and perpendicular to the top surface **312c** of the outer circular projection **312.** While **Figs. 14** **and** **15** depict the lancet receiving element **310** as including two locking members **316,** it is understood that, in other embodiments, the lancet receiving element **310** may include more or less locking members **316** (e.g., 1, 3, 5, etc.). Each locking member **316** includes a hook **318** that extends inwardly from a top of a locking member **316** towards the inner circular projection **314.** As will be discussed in further detail herein, the hooks **318** of the locking members **316** couple to the lancet 100 to retain the lancet **100** within the base **300.**

With particular reference to **Fig. 16****,** the base **300** includes a physiological sample well 320 and a physiological sample channel **322** that extends from and is in open communication with the physiological sample well **320.** As will be discussed in further detail herein, the physiological sample channel **322** is a fluidic channel that is configured to carry a physiological sample extracted from a subject. The physiological sample well **320** and a portion of the physiological sample channel **322** are open with respect to the bottom surface **302b** of the bottom wall **302.** Stated another way, the physiological sample well **320** and a portion of the physiological sample channel **322** do not include a bottom surface. The physiological sample well **320** is in open communication with the needle aperture **310.** As will be discussed in further detail herein, when drawing a physiological sample, the needle(s) **110** of the lancet **100** extends through the needle aperture **308** and through the physiological sample well **320** to pierce the skin of the subject. The physiological sample channel **322** extends from the physiological sample channel **322** and through the bottom wall **302** of the base **300.**

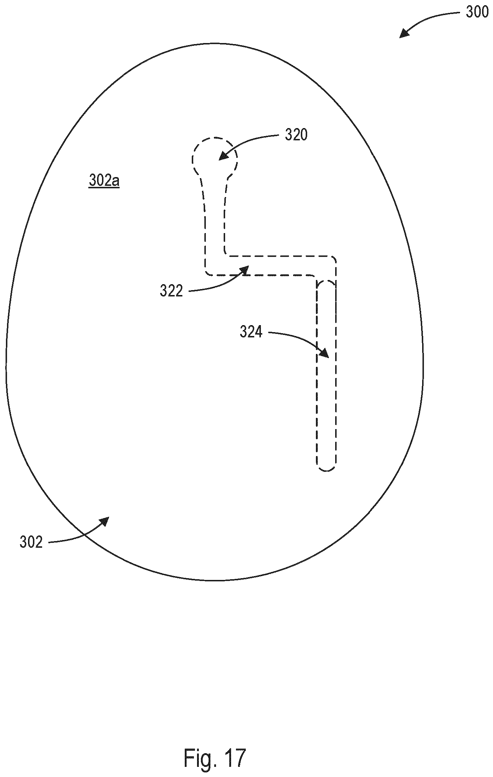

The bottom wall **302** of the base **300** further includes a sample collection reservoir **324 (****Fig. 17****)** that is configured to receive and store a drawn physiological sample. The sample collection reservoir **324** is in open communication with the physiological sample channel **322.** Accordingly, the physiological sample channel **322** carries a drawn physiological sample from the physiological sample well **320** to the sample collection reservoir **324.** The sample collection reservoir **324** is disposed vertically below the viewing aperture **206.** In one embodiment, the collection reservoir **324** is covered by a transparent material which allows a user of the dermal patch system **10** to view the sample collection reservoir **324** via the viewing aperture **206.** In some embodiments, a sample collection pad (e.g., a CF12 collection pad) is disposed within the sample collection reservoir **324** and absorbs the drawn physiological sample.

The base **300** includes an adhesive layer (not shown) disposed on the bottom surface **302b** of the bottom wall **302.** The cartridge **12** can be attached to the skin of a subject via the adhesive layer. The cartridge **12** may be attached anywhere on the subject's skin that is capable of supporting the cartridge **12** (e.g., on a leg, arm, etc. of the subject). The adhesive layer includes an opening that surrounds the physiological sample well **320** and through which the needle(s) **110** can extend to puncture the subject's skin. The adhesive layer covers and therefore seals the open portion of the physiological sample channel.

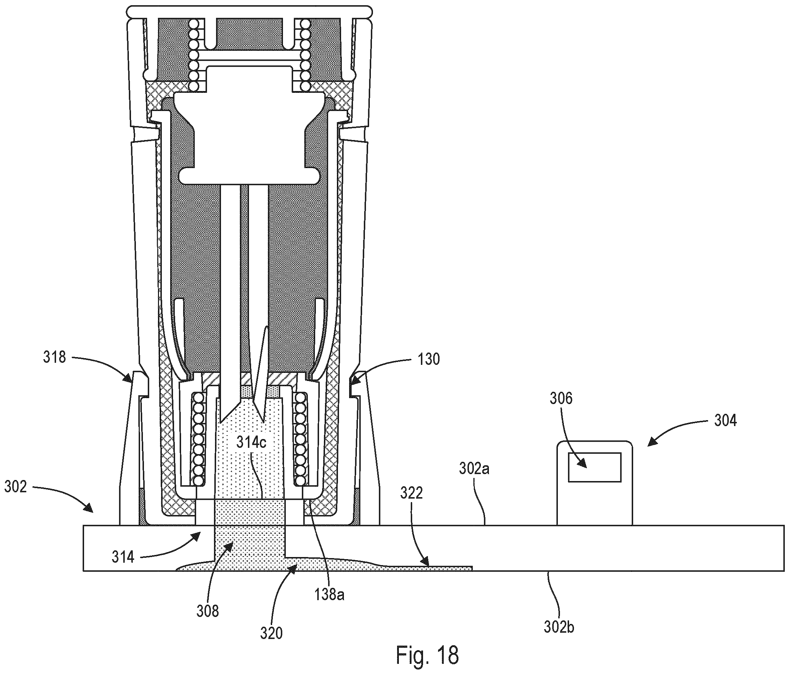

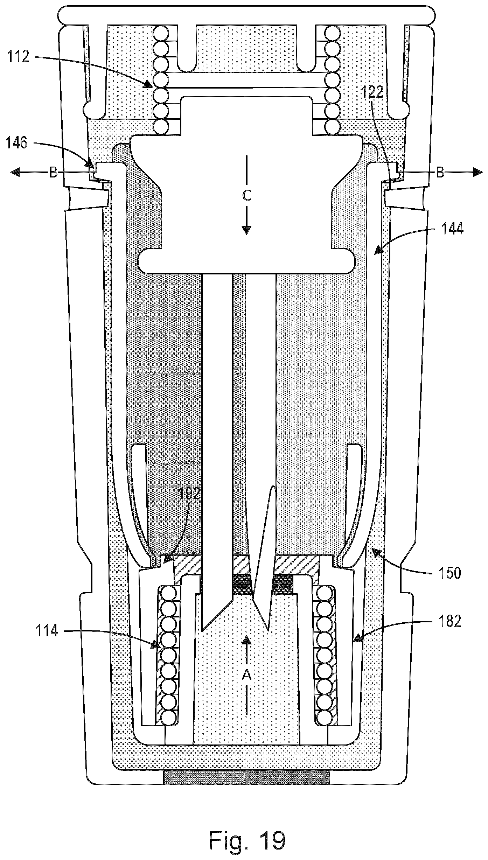

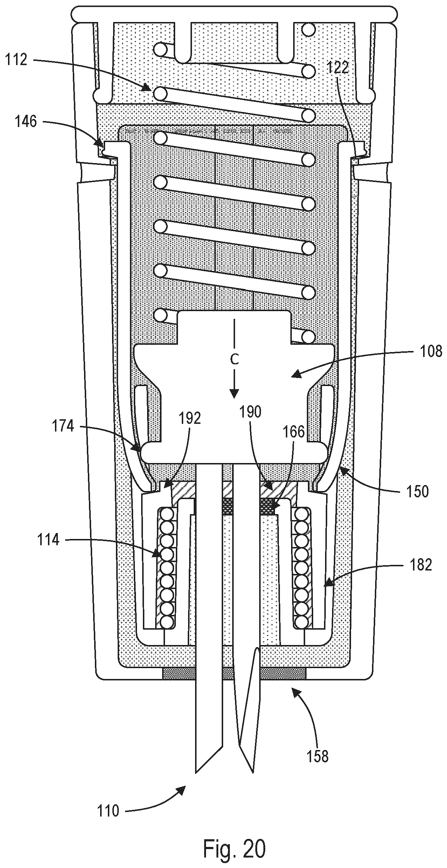

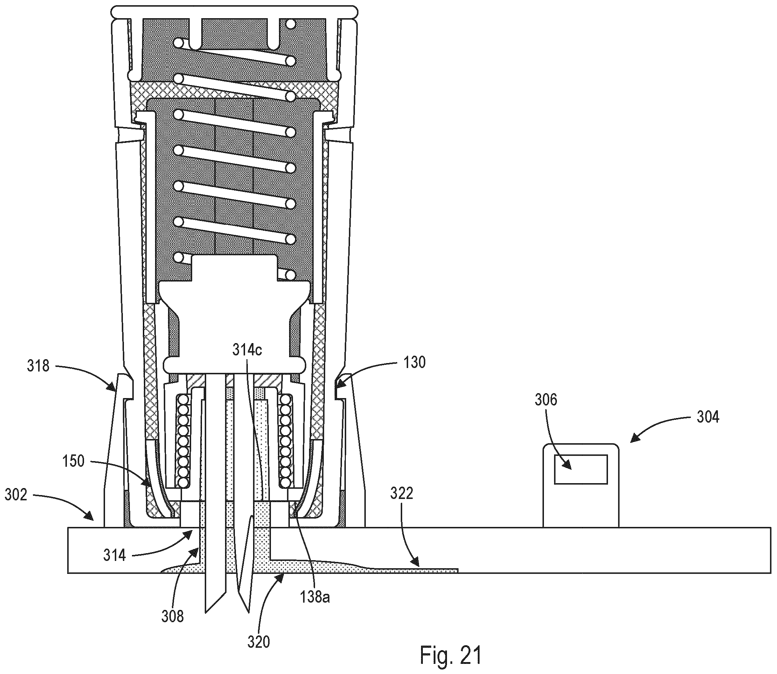

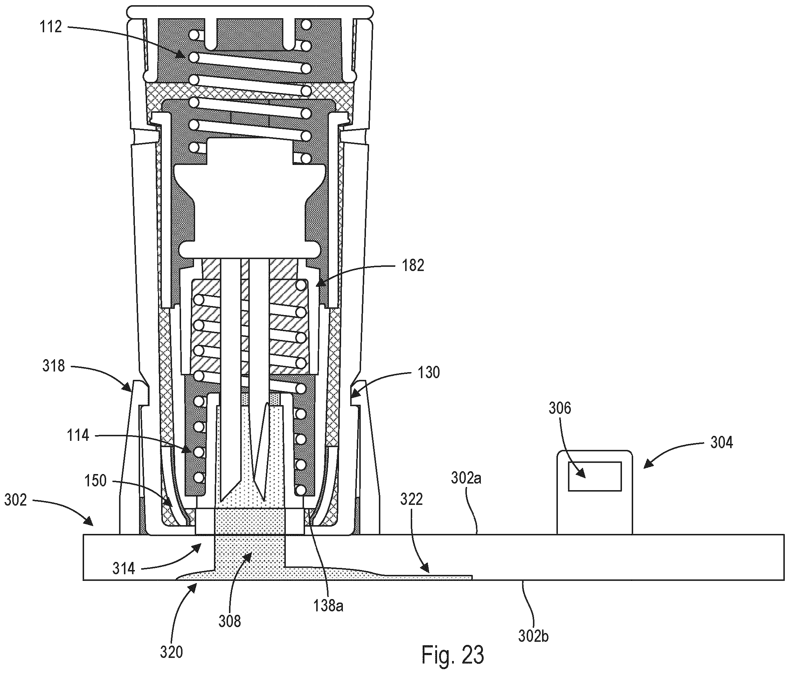

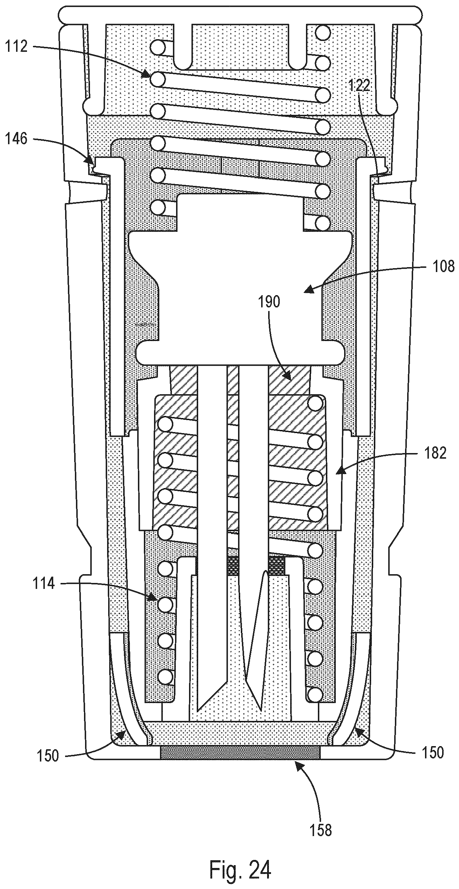

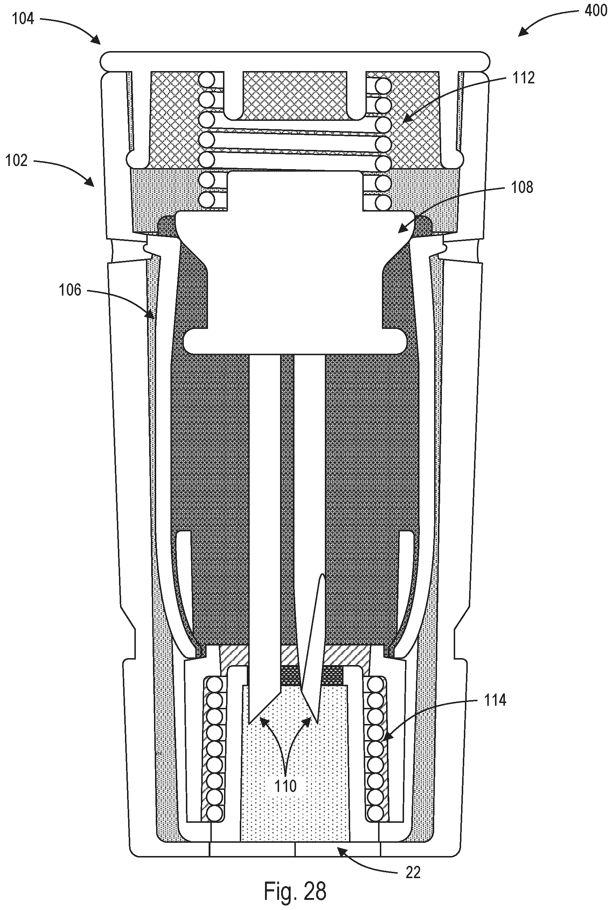

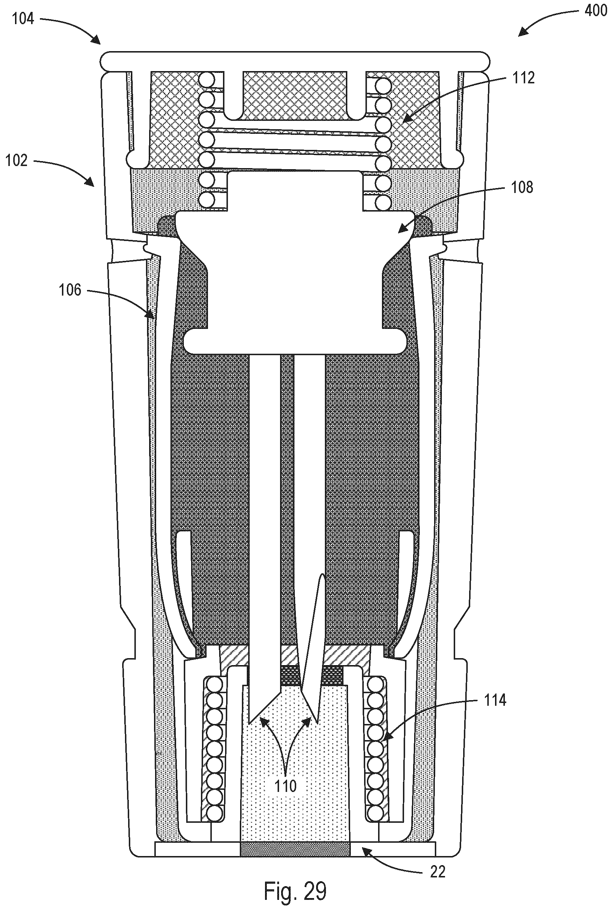

The lancet 100 is moveable between an undeployed position **(****Fig. 18** and **Fig. 19****),** a deployed position **(****Figs. 20****,** **21** and **22****),** and a retracted position **(****Figs. 23** and **24****).** A fully retracted position is shown in **Fig. 28** and **Fig. 29****.**

In the undeployed position, the hooks **146** (see **FIG. 8****)** of the inner sleeve **106** extend through the openings **126** of the housing **102,** which retains the lancet **100** in the undeployed position and prevents the lancet **100** from moving to the deployed position. In this position, the extensions **144** are compressed inward toward the center of the lancet **100** which allows the needle platform **108** to rest upon the inner sleeve **106** thereby preventing the needle platform from moving vertically downward and into the deployed position. Specifically, the angled surface **178** of the extensions **176** of the needle platform **108** contacts rests upon the extensions **144.**

Furthermore, the injection spring **112** extends around the body **170** of the needle platform **108** and the outer surface **136a** of the inner cylinder **136.** The injection spring **112** extends vertically between the inner surface **132b** of the top wall **132** of the cap **104** and the upper circular extension **172** of the needle platform **108.** In the undeployed position, the injection spring **112** is compressed between the top wall **132** of the cap **104** and the upper circular extension **172** of the needle platform **108.** The retraction spring **114** extends around the post **160** of the inner sleeve **106.** The retraction spring **114** extends vertically between the bottom wall **140** of the inner sleeve **106** and the top wall **186** of the spring sleeve **182.** That is, the retraction spring **114** extends vertically between the inner surface **140b** of the bottom wall **140** of the inner sleeve **106** and the inner surface **186b** of the top wall **186** of the spring sleeve **182.** When the lancet **100** is in the undeployed position, the retraction spring **114** is compressed between the bottom wall **140** of the inner sleeve **106** and the top wall **186** of the spring sleeve **182.** Furthermore, in the undeployed position, the deformable tabs **150** contact the ledge **192** of the spring sleeve **182** so as to prevent the retraction spring from expanding and moving the spring sleeve **182.**

The lancet **100** may be coupled to the cartridge **12** by pushing the lancet **100** through the lancet aperture **208** and into the base **300.** This engagement causes the needle(s) **110** of the lancet **100** to automatically move from an undeployed position to a deployed position and automatically from the deployed position to a retracted position. Furthermore, when the lancet **100** engages with the base **300,** the hooks **318** of the locking members **316** couple to the groove **130** of the housing **102** via a snap fitting. This coupling causes the generation of an audible clicking sound that indicates to a user that the lancet **100** was correctly inserted into the cartridge **12.**

As depicted in **Figs. 18****,** **21****, and** **23****,** when the lancet **100** is coupled to the base **300** the inner circular projection **314** of the lancet receiving element **310** extends through the aperture **128** of the housing **102** and contacts the bottom wall **140** of the inner sleeve **106.** Specifically, the top surface **314c** of the inner circular projection **314** contacts the outer surface **140a** of bottom wall **140** which forces the inner sleeve **106** to move vertically upward in the direction of arrow **A** within the housing **102.** As previously discussed herein, grooves **124** of the housing **102** and the protrusions **154** of the inner sleeve **106** guide the vertical movement of the inner sleeve **106.** The vertical movement of the inner sleeve **106** causes the hooks **146** to disengage from the openings **126** and causes the extensions **144** to extend vertically above and rest upon the ledge **122.** In this position, the extensions **144** decompress and expand outwardly in the direction of arrow **B.**

When the extensions **144** expand, the needle platform **108** no longer rests upon the extensions **144** and hence can move vertically downward in the direction of arrow **C** (see **Fig. 20****).** As the needle platform **108** moves downward, the injection spring **112** is allowed to expand and the force applied by the injection spring **112** causes the needle platform **108** (and therefore the needle(s) **110)** to travel with at a rate that is sufficient to cause the needle(s) **110** to puncture the skin of a subject wearing the dermal patch system **10.** The expansion of the injection spring **112** causes the needle(s) **110** to move and extend through the spring sleeve **182** via the opening **190,** through the post **160** via the opening **166,** through the opening **158** of the inner sleeve **106** and exit the housing **102** via the aperture **128** and pass through the base **300** of the cartridge **12** via the needle aperture **308.** Stated another way, the injection spring **112** moves the needle(s) **110** to the deployed position.

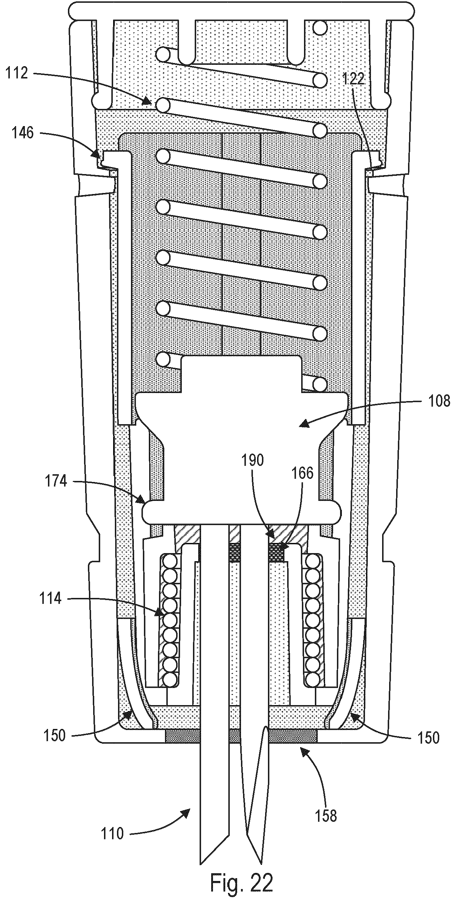

As previously discussed herein the grooves **156** of the inner sleeve **106** and the projections **180** of the needle platform **108** guide the vertical movement of the needle platform **108.** As the needle platform moves in the direction of arrow **B,** the lower circular extension **174** contacts the deformable tabs **150 (****Fig. 20****).** This contact causes the tabs **150** to deflect away from the spring sleeve **182** of the lancet **100** or causes the tabs **150** to break. As depicted in **Fig. 22****,** when the tabs **150** break, the broken piece(s) of the tabs **150** fall into a void between the inner sleeve **106** and the housing **102.** In the deployed position, the needle(s) **110** extend beyond the housing **102** and the lower circular extension **174** contacts the top wall **186** of the spring sleeve **182.**

When the tabs **150** deflect away from the spring sleeve **182** or break, the tabs **150** no longer contact the ledge **192** of the spring sleeve **182.** As a result, the retraction spring **114** is allowed to expand. The expansion of the retraction spring **114** moves the spring sleeve **182** vertically upward in the direction of arrow **D.** If the tabs **150** deflect and do not break, the spring sleeve **182** prevents the tabs **150** from contacting the retraction spring **114** as the retraction spring **114** expands. The spring sleeve **182** moves the needle platform **108** in the direction of arrow **D** to place the lancet **100** in the retracted position. That is, after moving to the deployed position, the retraction spring **114** causes the needle(s) **110** to retract back into the inner volume **142** of the inner sleeve **106** via the needle aperture **308** of the base **300,** the aperture **128** of the housing **102,** and the opening **158** of the inner sleeve **106.** That is, after penetrating the skin of a subject, the retraction spring **114** causes the needle(s) **110** to automatically retract back into the housing of the lancet **100** thereby placing the lancet **100** in the retracted position.

In this embodiment, the three needles **110** have substantially the same length, and by simultaneously moving the three needles **110** to the deployed position, the lancet **100** punctures the skin of the subject at three locations at the same time, though in other embodiments, two or all of the three needles **110** may have different lengths.

In some embodiments, the use of three needles **110** results in placing a portion of the skin under tension, which can in turn facilitate drawing blood through on or more punctures caused by the needles. In particular, as noted above, it has been discovered that the use of three needles **110** can advantageously allow a larger volume of blood to be drawn compared to the use of one, two, or a greater number of needles **110.** The invention is not limited to three needles. One, two, three or more needles can be used. Without being limited to any particular theory, in some embodiments, in addition to placing a skin portion under tension, the use of three needles **110** may also improve the likelihood that at least one of the needles would cut through a capillary to extract a blood volume from the subject. This is because when the skin is tensioned, one of the needles can penetrate the skin at a different depth. As a result, it is possible to have a higher volume of blood flow out of one of the punctures relative to the other two. Also, when three needles are used, one of the needles can be used to tent the skin to improve the likelihood of puncturing the skin. Each of the needles **110** can has a tapered tip that points outward (away from the center of the lancet) to maximize a distance between puncture locations on the subject's skin. Having a greater distance between puncture points can increase the likelihood that a capillary is targeted, thereby increasing the probability of bleeding. Furthermore, the three needles **110** may be positioned such that the needle tips form an equilateral triangle and are therefore equally space from one another.

It is understood that the lancet **100** may be used with any cartridge of a dermal patch system that is configured to engage with the lancet **100.** That is, the lancet **100** may be used with any cartridge that includes an extension that causes the lancet **100** to transition from the undeployed position to the deployed position.

After the needles **110** retract, a physiological sample pools within the physiological sample well **320** of the base **300.** Due to gravity, wicking, and capillary action, the physiological sample travels from the physical sample well **320** to the sample collection reservoir **324** via the physiological sample channel **322.** A user can view the sample collection reservoir **324** via the viewing aperture **206** to ensure the physiological sample has traveled to the sample collection reservoir **324.** After drawing the physiological sample, the cartridge **12** may be removed from the skin of the subject and sent to a laboratory for analysis. A medical professional may then extract the physiological sample and analyze the sample.

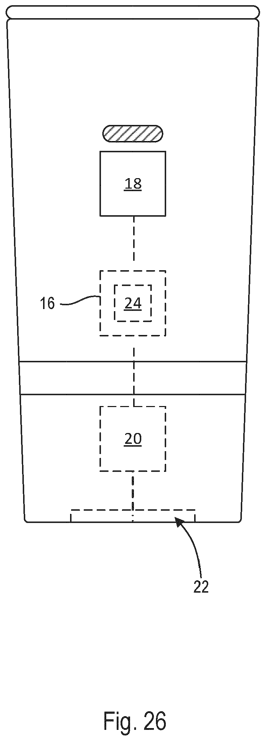

As shown in **Fig. 26****,** the lancet 400 can include a sensor **18** that can create a digital image of the fingerprint of the user of the lancet and electronics **16** that process the digital image. The sensor 18 can be a fingerprint sensor, or any type of biometric sensor that can identify the user of the lancet. For example, a finger image sensor (FIS), or a contact image sensor (CIS), such as the M116-A8CIU sensor or the M116-A6C1P sensor made by CMOS Sensor Inc. in Cupertino, CA, can be used.

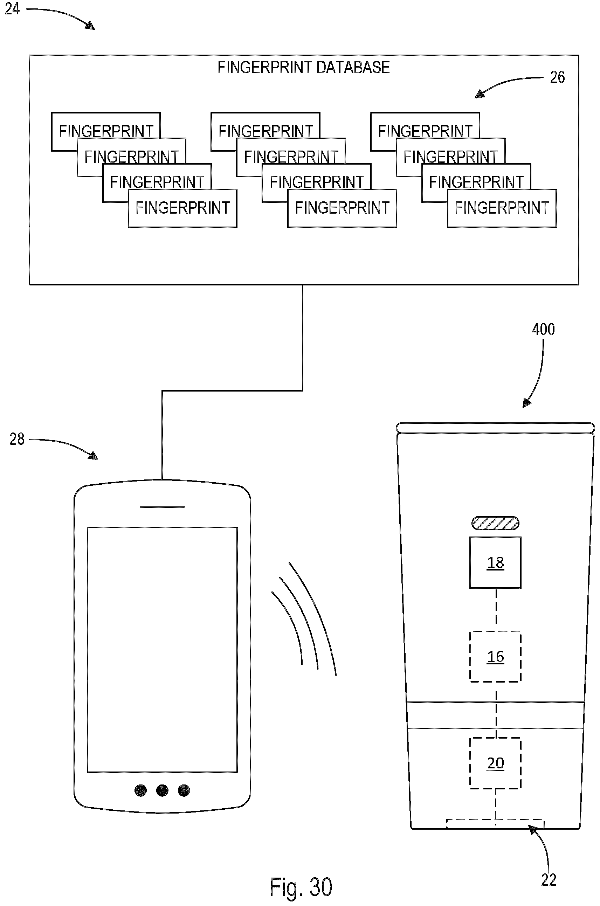

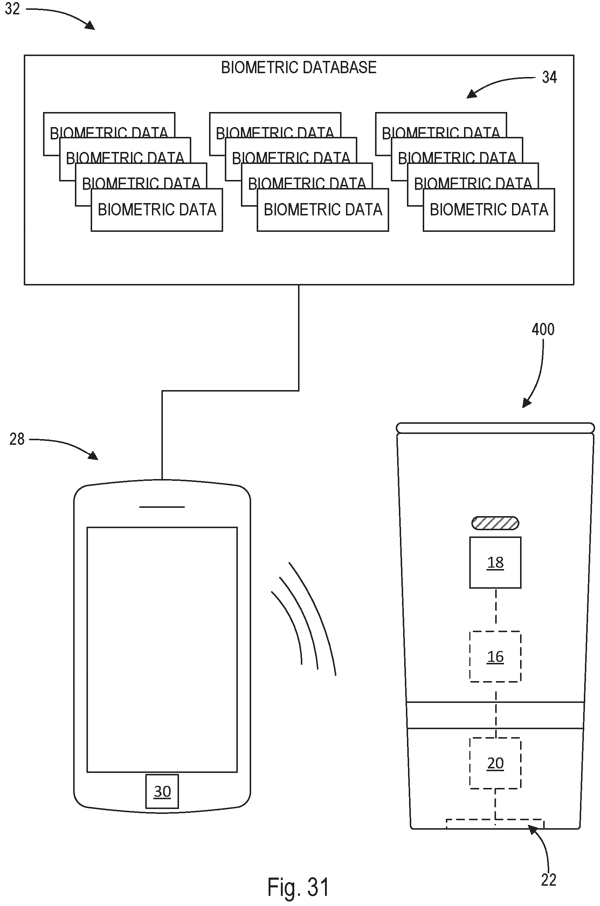

In another embodiment **(****Fig. 30****),** a first computer system **28** is connected to a fingerprint database **24** that includes data for a plurality of fingerprints **26,** each associated with a different subject. In this embodiment, before drawing a physiological sample from a subject, the computer system **28** receives via Bluetooth, or other wireless communication method, the fingerprint image output by the sensor **18** on the lancet **400.** As described above, the sensor **18** can scan the fingerprint of the finger that is applied to the lancet **400** and produce a digital image of the user's fingerprint. The computer system **28** then can identify the subject by comparing the digital image of the user's fingerprint with the fingerprint data **26.** The identity of the subject can then be associated with the QR code on the cartridge **12.** While the above describes a system that identifies a subject based on a fingerprint, the system may identify a subject from which a physiological sample has been drawn based on any database that includes a subject's identity, such as the biometric database **32** shown in **Fig. 31****.**

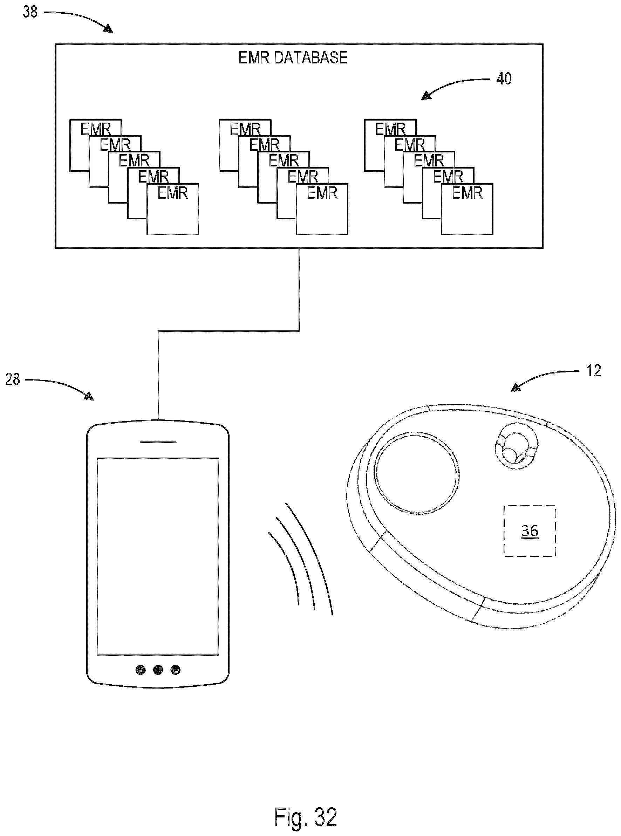

In another embodiment **(****Fig. 32****),** a first computer system **28** is connected to an electronic medical record ("EMR") database **38** that includes a plurality of EMR's **40** each associated with a different subject. In this embodiment, after drawing a physiological sample from a subject, the computer system **28** scans the QR code **36** on the cartridge **12** and sends the cartridge **12** to a laboratory for further analysis. In response to scanning the QR code **36,** computer system **28** associates the QR code **36** with an EMR **40** that corresponds to the subject from which the physiological sample was drawn. In some embodiments, the computer system **28** may update the associated EMR **40** to indicate a physiological has been drawn automatically or based on a user input. While the above describes a system for identifying a subject from which a physiological sample has been drawn by matching QR codes in an EMR database, the system may identify a subject from which a physiological sample has been drawn by matching the physiological sample to any database that includes a subject's identity, such as the fingerprint database shown in **Figs.** 30 and the biometric database shown in **Fig. 31****.**

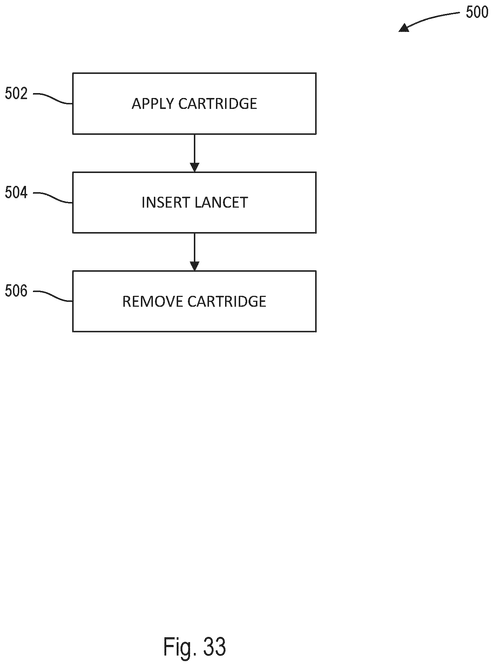

Referring now to **Fig. 33****,** a method **500** for obtaining a physiological sample from a subject is disclosed herein.

At **502,** a cartridge **12** of the dermal patch system **10** is affixed to the skin of a subject as previously discussed herein.

**At 504,** the lancet **100** is inserted into the cartridge **12** to draw a physiological sample (e.g., a blood sample, a sample of interstitial fluid, etc.) from the subject as previously discussed herein.

At **506,** the user removes the cartridge **12** from the skin of the subject as previously discussed herein.

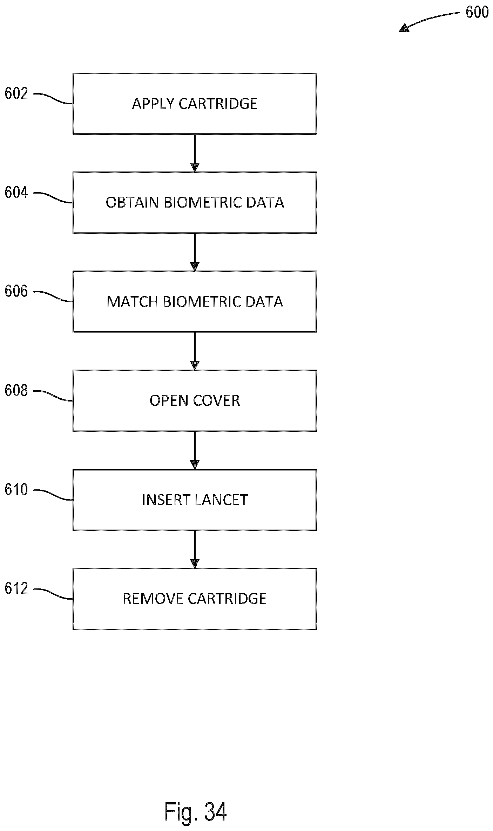

Referring now to **Fig. 34****,** a method **600** for obtaining a physiological sample from a subject is disclosed herein. At **602,** a user attaches the cartridge **12** of the dermal patch system **10** to the skin of a subject at a suitable location (e.g., arm, leg, etc.) as previously discussed herein. At **604,** a biometric sensor obtains biometric data from the user of the dermal patch system **10** and sends a signal indicative of the biometric data to the computer system **28** as previously discussed herein. At **606,** in response to receiving the signal indicative of the biometric data, the computer system determines whether the biometric data matches the biometric data stored in the memory of the computer system. At **608,** the cover **200** of the cartridge **12** is opened to allow access of the lancet into the cartridge 12. At **610,** the lancet **100** is inserted in the cartridge **12** to engage with the cartridge **12** to draw the physiological sample. At **612,** the cartridge **12** is removed from the skin of the subject as previously discussed herein. The cartridge **12** can then be sent to a medical professional to analyze the drawn physiological sample as previously discussed herein.

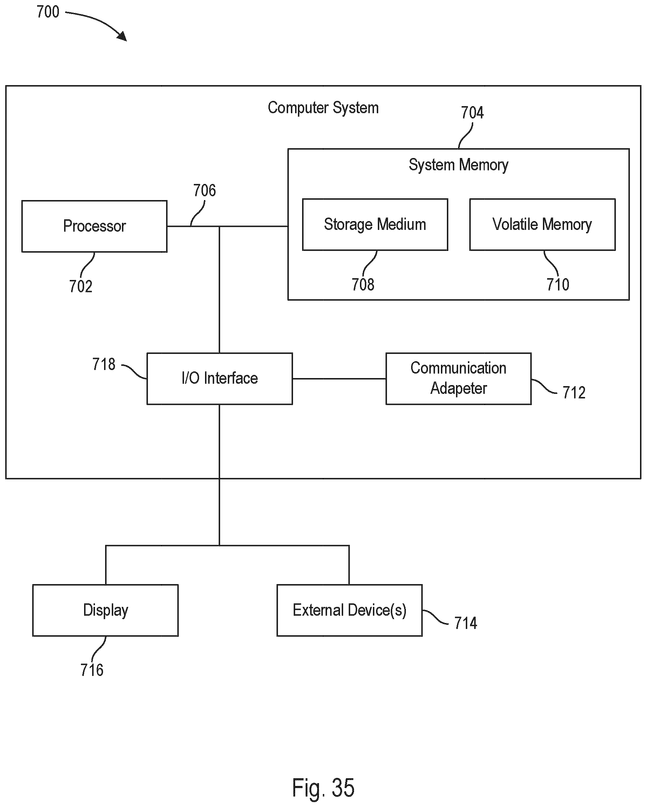

Referring now to **Fig. 35****,** a computer system **700** is shown in accordance with an exemplary embodiment. The computer system **700** may serve as any computer system disclosed herein (e.g., the computer system **28** in **Figs. 30****,** **31** **and** **32****).** As used herein a computer system (or device) is any system/device capable of receiving, processing, and/or sending data. Computer systems include, but are not limited to, microprocessor-based systems, personal computers, servers, hand-held computing devices, tablets, smartphones, multiprocessor-based systems, mainframe computer systems, virtual reality ("VR") headsets and the like.

As shown in **Fig. 35****,** the computer system **700** includes one or more processors or processing units **702,** a system memory **704,** and a bus **706** that couples the various components of the computer system **700** including the system memory **704** to the processor **702.** The system memory **704** includes a computer readable storage medium **708** and volatile memory **710** (e.g., Random Access Memory, cache, etc.). As used herein, a computer readable storage medium includes any media that is capable of storing computer readable; program instructions and is accessible by a processor. The computer readable storage medium **708** can include non-volatile and non-transitory storage media (e.g., flash memory, read only memory (ROM), hard disk drives, etc.). Computer program instructions as described herein include program modules (e.g., routines, programs, objects, components, logic, data structures, etc.) that are executable by a processor. Furthermore, computer readable program instructions, when executed by a processor, can direct a computer system to function in a particular manner such that a computer readable storage medium comprises an article of manufacture. Specifically, the computer readable program instructions when executed by a processor can create a means for carrying out at least a portion of the steps of the methods disclosed herein.

The bus **706** may be one or more of any type of bus structure capable of transmitting data between components of the computer system **700** (e.g., a memory bus, a memory controller, a peripheral bus, an accelerated graphics port, etc.).

The computer system **700** may further include a communication adapter **712** which allows the computer system **700** to communicate with one or more other computer systems/devices via one or more communication protocols (e.g., Wi-Fi, BTLE, etc.) and in some embodiments may allow the computer system **700** to communicate with one or more other computer systems/devices over one or more networks (e.g., a local area network (LAN), a wide area network (WAN), a public network (the Internet), etc.).

In some embodiments, the computer system **700** may be connected to one or more external devices **714** and a display **716.** As used herein, an external device includes any device that allows a user to interact with a computer system (e.g., mouse, keyboard, touch screen, etc.). An external device **714** and the display **716** may be in communication with the processor **702** and the system memory **904** via an Input/Output (I/O) interface **718.**

The display **716** may display a graphical user interface (GUI) that may include a plurality of selectable icons and/or editable fields. A user may use an external device **714** (e.g., a mouse) to select one or more icons and/or edit one or more editable fields. Selecting an icon and/or editing a field may cause the processor **702** to execute computer readable program instructions stored in the computer readable storage medium **708.** In one example, a user may use an external device **714** to interact with the computer system **700** and cause the processor **702** to execute computer readable program instructions relating to at least a portion of the steps of the methods disclosed herein.

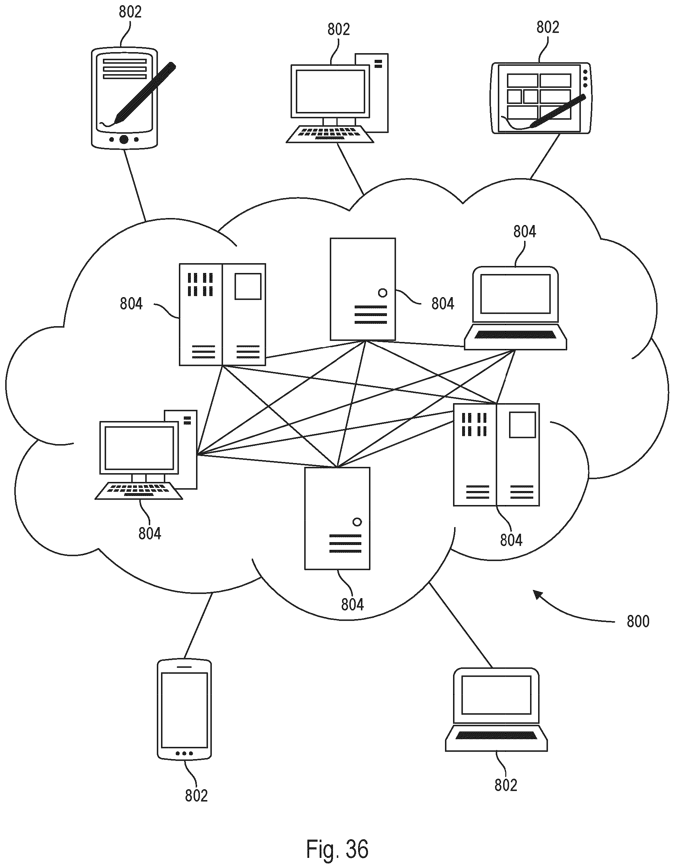

Referring now to **Fig. 36****,** a cloud computing environment **800** is depicted in accordance with an exemplary embodiment. The cloud computing environment **800** is connected to one or more user computer systems **802** and provides access to shared computer resources (e.g., storage, memory, applications, virtual machines, etc.) to the user computer systems **802.** As depicted in **Fig. 36****,** the cloud computing environment includes one or more interconnected nodes **804.** Each node **804** may be a computer system or device local processing and storage capabilities. The nodes **804** may be grouped and in communication with one another via one or more networks. This allows the cloud computing environment **800** to offer software services to the one or more computer services to the one or more user computer systems **802** and as such, a user computer system **802** does not need to maintain resources locally.

In one embodiment, a node **804** includes computer readable program instructions for carrying out various steps of various methods disclosed herein. In these embodiments, a user of a user computer system **802** that is connected to the cloud computing environment may cause a node **804** to execute the computer readable program instructions to carry out various steps of various methods disclosed herein.

As previously discussed, the above may be implemented by way of computer readable instructions, encoded or embedded on computer readable storage medium (which excludes transitory medium), which, when executed by a processor(s), cause the processor(s) to carry out the methods of the present disclosure.

While various embodiments have been illustrated and described in detail in the drawings and foregoing description, such illustration and description are to be considered illustrative or exemplary and not restrictive; embodiments of the present disclosure are not limited to the disclosed embodiments. Other variations to the disclosed embodiments can be understood and effected by those skilled in the art in practicing embodiments of the present disclosure, from a study of the drawings, the disclosure, and the appended claims.

In the claims, the word "comprising" does not exclude other elements or steps, and the indefinite article "a" or "an" does not exclude a plurality. A single processor or other processing unit may fulfill the functions of several items recited in the claims. The mere fact that certain measures are recited in mutually different dependent claims does not indicate that a combination of these measured cannot be used to advantage. Any reference signs in the claims should not be construed as limiting the scope. The disclosure also comprises the following embodiments:
1. A dermal patch system comprising:
   a lancet including one or more needles, and
   a cartridge configured to attach to the skin of a subject,
      wherein the lancet is configured automatically move the one or more needles from an undeployed position to a deployed position when the lancet engages with the cartridge,
      wherein theone or more needles are configured to puncture the skin of the subject to draw a physiological sample when in the deployed position.
2. The dermal patch system of embodiment 1, wherein the cartridge is configured to store the drawn physiological sample.
3. The dermal patch system of embodiment 1, wherein the one or more needles includes two needles.
4. The dermal patch system of embodiment 1, wherein the one or more needles includes three needle.
5. The dermal patch system of embodiment 1, wherein the lancet includes a housing, and the one or more needles are disposed within the housing when in the undeployed position and extend beyond the housing when in the deployed position.
6. The dermal patch system of embodiment 5, wherein the one or more needles are configured to automatically retract into the housing after puncturing the skin.
7. The dermal patch system of embodiment 6, wherein the lancet further includes:
   an injection spring configured to move the one or more needles to the deployed position, and
   a retraction spring configured to retract the one or more needles into the housing.
8. The dermal patch system of embodiment 7, further comprising a mechanism for maintaining the retraction spring is a compressed state when the one or more needles are in the undeployed position and is configured to expand to retract the one or more needles.
9. The dermal patch system of embodiment 7, wherein the lancet further includes a plurality of deformable tabs configured to compress the retraction spring when the one or more needles are in the undeployed position.
10. The dermal patch system of embodiment 9, wherein the lancet further includes:
   a needle platform that carries the one or more needles from the unemployed position to the deployed position,
   wherein the needle platform is configured to deform or break the tabs when the lancet engages with the cartridge, thereby allowing the retraction spring to expand and cause the one or more needles to deploy.
11. The dermal patch system of embodiment 10, wherein the needle platform is configured to break the tabs.
12. A method for obtaining a physiological sample from a subject, comprising:
   affixing a cartridge of a dermal patch system to the skin of a subject,
   engaging a lancet with the cartridge, wherein the lancet includes one or more needles,
      wherein engaging the lancet with the cartridge automatically moves the one or more needles from an undeployed position to a deployed position thereby drawing a physiological sample.
13. The method of embodiment 12, further comprising:
   storing physiological sample within the cartridge.
14. The method of embodiment 12, further comprising:
   retracting the one or more needles into the lancet, and
   removing the cartridge from the skin of the subject.
15. The method of embodiment 14, wherein the lancet automatically retracts the one or more needles.
16. The method of embodiment 15, wherein the lancet includes a first spring that moves the one or more needles to the deployed position and a second spring that retracts the needles.
17. The method of embodiment 16, further comprising:
   deforming a portion of the lancet whole moving the one or more needles to the deployed position.
18. The method of embodiment 17, wherein deforming a portion of the lancet allows the second spring to retract the one or more needles.
19. The dermal patch system of embodiment 12, wherein the one or more needles includes two needles.
20. The dermal patch system of embodiment 12, wherein the one or more needles includes three needles.
21. The dermal patch system of embodiment 1, wherein the one or more needles includes four needles.
22. The dermal patch system of embodiment 4, wherein the one or more needles are equally spaced from one another.
23. The dermal patch system of embodiment 22, wherein tips of the three needles form an equilateral triangle.
24. The dermal patch system of embodiment 12, wherein the one or more needles includes four needles.
25. The dermal patch system of embodiment 20, wherein the one or more needles are equally spaced from one another.
26. The dermal patch system of embodiment 25, wherein tips of the three needles form an equilateral triangle.

## Claims

1. A dermal patch system comprising:
a lancet including one or more needles, and
a cartridge configured to attach to the skin of a subject,
wherein the lancet is configured automatically move the one or more needles from an undeployed position to a deployed position when the lancet engages with the cartridge,
wherein the one or more needles are configured to puncture the skin of the subject to draw a physiological sample when in the deployed position.

2. The dermal patch system of claim 1, wherein the cartridge is configured to store the drawn physiological sample.

3. The dermal patch system of claim 1, wherein the one or more needles includes two or more needles.

4. The dermal patch system of claim 1, wherein the lancet includes a housing, and the one or more needles are disposed within the housing when in the undeployed position and extend beyond the housing when in the deployed position.

5. The dermal patch system of claim 4, wherein the one or more needles are configured to automatically retract into the housing after puncturing the skin.

6. The dermal patch system of claim 5, wherein the lancet further includes:
an injection spring configured to move the one or more needles to the deployed position, and
a retraction spring configured to retract the one or more needles into the housing.

7. The dermal patch system of claim 6, further comprising a mechanism for maintaining the retraction spring is a compressed state when the one or more needles are in the undeployed position and is configured to expand to retract the one or more needles.

8. The dermal patch system of claim 6, wherein the lancet further includes a plurality of deformable tabs configured to compress the retraction spring when the one or more needles are in the undeployed position.

9. The dermal patch system of claim 8, wherein the lancet further includes:
a needle platform that carries the one or more needles from the unemployed position to the deployed position,
wherein the needle platform is configured to deform or break the tabs when the lancet engages with the cartridge, thereby allowing the retraction spring to expand and cause the one or more needles to deploy.

10. The dermal patch system of claim 9, wherein the needle platform is configured to break the tabs.

11. A method for obtaining a physiological sample from a subject, comprising:
affixing a cartridge of a dermal patch system to the skin of a subject,
engaging a lancet with the cartridge, wherein the lancet includes one or more needles,
wherein engaging the lancet with the cartridge automatically moves the one or more needles from an undeployed position to a deployed position thereby drawing a physiological sample.

12. The method of claim 11, further comprising:
storing physiological sample within the cartridge.

13. The method of claim 11, further comprising:
retracting the one or more needles into the lancet, and
removing the cartridge from the skin of the subject.

14. The method of claim 13, wherein the lancet automatically retracts the one or more needles.

15. The dermal patch system of claim 1, wherein the one or more needles include three needles, and tips of the three needles form an equilateral triangle.
